# EUROPEAN PATENT SPECIFICATION

(11) **EP 1 904 637 B1**
(45) Date of publication and mention of the grant of the patent: **27.10.2010**
(21) Application number: 06765859.1
(22) Date of filing: 26.06.2006
(51) Int. Cl.: C12N 15/82, A01H 5/00

(54) **TRANSFORMED PLANTS ACCUMULATING MONO- AND/OR SESQUITERPENES**
TRANSFORMIERTE PFLANZEN MIT ANREICHERUNG VON MONO- UND/ODER SESQUITERPENEN
PLANTES TRANSFORMÉES PAR ACCUMULATION DE MONO- ET/OU DE SESQUITERPÈNES

(30) Priority: 01.07.2005 US 696116 P; 07.07.2005 EP 05106199
(43) Date of publication of application: 02.04.2008
(73) Proprietor: University of Kentucky Research Foundation, Lexington, KY 40506-0286 (US)
(72) Inventor: CHAPPELL, Joe, Lexington, Kentucky 40503 (US); WU, Shuiqin, Lexington, Kentucky 40503 (US); SCHALK, Michel, F-74160 Collonges-sous-saleve (FR); CLARK, Anthony, West Windsor, New Jersey 08550 (US)
(74) Representative: Salvaterra-Garcia, Maria de Lurdes
(86) International application number: PCT/IB2006/052076
(87) International publication number: WO 2007/004104

(56) References cited:
- WO-A-01/31027
- WO-A-2006/014837
- WO-A2-00/12725
- WO-A2-03/047547
- US-A1- 2004 072 323
- LUCKER JOOST ET AL: "Increased and altered fragrance of tobacco plants after metabolic engineering using three monoterpene synthases from lemon" PLANT PHYSIOLOGY, AMERICAN SOCIETY OF PLANT PHYSIOLOGISTS, ROCKVILLE, MD, US, vol. 134, no. 1, January 2004 (2004-01), pages 510-519, XP002355232 ISSN: 0032-0889
- JACKSON BETH E ET AL: "Metabolic engineering to produce sesquiterpenes in yeast." ORGANIC LETTERS, vol. 5, no. 10, 15 May 2003 (2003-05-15), pages 1629-1632, XP002420393 ISSN: 1523-7060
- MARTIN VINCENT J J ET AL: "Engineering a mevalonate pathway in Escherichia coli for production of terpenoids." NATURE BIOTECHNOLOGY, vol. 21, no. 7, July 2003 (2003-07), pages 796-802, XP002420804 ISSN: 1087-0156
- CHAPPELL JOSEPH ET AL: "Is the reaction catalyzed by 3-hydroxy-3-methylglutaryl coenzyme A reductase a rate-limiting step for isoprenoid biosynthesis in plants?" PLANT PHYSIOLOGY, AMERICAN SOCIETY OF PLANT PHYSIOLOGISTS, ROCKVILLE, MD, US, vol. 109, no. 4, 1995, pages 1337-1343, XP002133625 ISSN: 0032-0889
- AHARONI A ET AL: "Terpenoid metabolism in wild-type and transgenic Arabidopsis plants" PLANT CELL, AMERICAN SOCIETY OF PLANT PHYSIOLOGISTS, ROCKVILLE, MD, US, vol. 15, no. 12, December 2003 (2003-12), pages 2866-2884, XP002322003 ISSN: 1040-4651
- POLAKOWSKI T ET AL: "OVEREXPRESSION OF A CYTOSOLIC HYDROXYMETHYLGLUTARYL-COA REDUCTASE LEADS TO SQUALENE ACCUMULATION IN YEAST" APPLIED MICROBIOLOGY AND BIOTECHNOLOGY, SPRINGER VERLAG, BERLIN, DE, vol. 49, 1998, pages 66-71, XP001167014 ISSN: 0175-7598
- RO DAE-KYUN ET AL: "Production of the antimalarial drug precursor artemisinic acid in engineered yeast" NATURE (LONDON), vol. 440, no. 7086, April 2006 (2006-04), pages 940-943, XP002420805 ISSN: 0028-0836

## Description

### Technical Field

The present invention relates to transformed plants. In particular, transformed plants that accumulate mono- and/or sesquiterpenes. The plants are transformed to over-express or to comprise additional genes encoding a HMG-CoA reductase (HMGR) and a terpene synthase (TS), and, optionally, a prenyltransferase (PRT), these genes being nuclear genes devoid of a plastid targeting sequence. The invention further relates to methods for preparing the plants, a method for altering the content of a specific terpene in a plant, a method for producing a terpene and the use of the above-mentioned genes for producing transformed plants.

### Background of the Invention and Problem to be Solved

Terpenes and terpenoids are found in most organisms. Their important commercial value, which is constantly increasing, is linked to the diverse range of bioactivities and functionalities encompassed by different terpenes. Accordingly, many vitamins, hormones, insect repellents, drugs, flavors and fragrances are found amongst this very large class of compounds, which all are made starting from units of 5 carbons called isoprene units.

Terpenes can be classified by the number of isoprene units present in their structure: monoterpenes (C₁₀), sesquiterpenes (C₁₅), diterpenes (C₂₀), triterpenes (C₃₀), tetraterpenes (C₄₀) and polyterpenes (Cₙ, n≥45). The plant kingdom contains a high diversity of mono- and sesquiterpenes representing thousands of different structures.

The chemical synthesis of higher terpenes such as sesquiterpenes is very complex and environmentally acceptable processes for their preparation have not yet been realized. Therefore, it is a first objective of the present invention to provide methods for efficiently producing or accumulating specific terpenes while avoiding multiple-step chemical synthesis.

Studies on the biosynthetic pathway of terpenes revealed that the common C₅-precursor to all terpenes is isopentenyl diphosphate (IPP). Two distinct pathways for IPP biosynthesis coexist in the plants. The mevalonate pathway (MVA) is found in the cytosol in association with the endoplasmic reticulum and the non-mevalonate pathway, also called deoxyxylulose or methyl-D-erythritol phosphate pathway (DOXP/MEP) is found in the plastids of higher plants. The starting products, the enzymes involved and the catalysed reactions are different in both pathways, and, in the cells of higher plants they operate in parallel and complement each other. Accordingly, the MVA pathway in the cytoplasm is responsible for the biosynthesis of sterols, sesquiterpenes, and polyterpenes, whereas the plastid (MEP pathway) provides C₅-units for the synthesis of monoterpenes, diterpenes, for example kaurene (C₂₀), and polyterpenes, for example carotenoids (C₄₀) and plastoquinone-9 (C₄₅).

Following the synthesis of IPP, it is repetitively condensed by prenyl transferases (PRT) to form the acyclic prenyl diphosphate terpene precursors for each class of terpenes, that is, geranyl-diphosphate (GPP) for the monoterpenes and farnesyl-diphosphate (FPP) for the sesquiterpenes. These precursors in turn serve as substrate for the terpene synthases or cyclases, which are specific for each class of terpene, e.g. monoterpene, and sesquiterpene synthases. Terpene synthases can catalyze complex multiple step cyclizations to form the large diversity of carbon skeleton of the terpene compounds.

Attempts have been made to isolate specific terpene synthases and WO2004/031376 reports the isolation of the genes encoding cubebol, valencene and germacrene synthases. When *E. coli* cells were transformed with plasmids containing these genes, the corresponding fragrance compounds could be found in the cultivating medium. Generally, in view of prior art concerned with heterologous expression of terpene synthases, it is an objective to provide means and methods for accumulating specific terpenes in still higher amounts.

In US 5,589,619, US 5,365,017, US 5,349,126 and US 5,349,126 processes for increasing squalene and sterol accumulation in transgenic plants are disclosed. These references, however, are silent as to how the accumulation of other classes of terpenes, such as mono- and sesquiterpenes could be increased.

The preparation of transgenic plants is also the subject of US 6,841,717, which relates to genes associated with the MEP-pathway. This reference teaches a DNA molecule encoding an HMBPP-Synthase (GCPE protein), which was linked to a chloroplast transit peptide and was thus used to produce a transgenic plant. While this reference deals with the accumulation of tocopherol substrates, it is silent how other terpene compounds can effectively be accumulated.

The present inventors address the problem of producing or accumulating a specific, selected terpene. Preferably, a method is provided, which is suitable to produce not only a pre-determined, but any terpene of interest The objective is thus to provide a system which allows, for example, the accumulation of any of the above-indicated sesquiterpenes, such as cubebol, valencene, germacrene, patchoulol, but which is also suitable to accumulate other terpenes, in particular monoterpenes. This problem has so far not been solved by the prior art, the latter basically suggesting recombinant organisms having modified properties in the MVA or MEP pathway, and observing that certain terpene end products get accumulated.

A further objective of the present invention is to provide means for generating any selected terpene, preferably a sesquiterpene, in a stereochemically pure form and with a reliable and cost effective production platform.

### Summary of the Invention

Remarkably, the present inventors successfully transformed organisms to express a terpene synthase (TS) and an un-regulated form of HMG-CoA reductase (HMGR) and obtained high yields of the terpene that is synthesised by the TS. Surprisingly, in plants transformed with a heterologuous, TS encoding gene alone, terpene accumulation was detectable but was modest and not correlated with TS protein expression levels. An important advantage of the present invention is that any desired terpene can be accumulated in any plant, if the nucleotide sequence encoding the synthase of the selected terpene is known or can routinely be isolated. This is possible by targeting preferably to the cytosol of a plant at least two gene products, that is a HMGR and the enzyme capable of synthesising the terpene, a TS.

Accordingly, the present invention provides, in a first aspect, a plant comprising and expressing a transgene encoding a HMG-CoA reductase (HMGR), a transgene encoding a terpene synthase (TS) and a transgene encoding a prenyltransferase (PRT), in which the genes encoding the HMGR, the TS and the PRT are nuclear genes devoid of a plastid targeting sequence.

In a second aspect, the present invention provides a method for preparing a transformed plant, the method comprising the steps of
- transforming plant material to comprise additional genes encoding a HMGR, a TS and a PRT, said genes being nuclear genes devoid of a plastid targeting sequence; and
- regenerating transformed plants from said plant material.

In a further aspect, the present invention provides a method for producing a terpene, the method comprising the step of isolating the terpene from the plant of the invention or obtainable by the method for preparing a transformed plant.

### Brief Description of the Drawings

In the figures,
**Figures 1A, 1B****, 1C and 1D** show, in part A and B, a GC-MS analysis of a control tobacco line (part B), and one transformed with both: transgenes encoding a HMG-CoA reductase (HMGR) and a sesquiterpene (patchoulol) synthase (PTS), part A. Peak 10 in chromatogram A is analysed by MS in part C and could be identified as patchoulol by comparison to authentic patchoulol (D). Peak 3 was used as an internal standard (3-α-cedrene).
**Figure 2** quantitatively shows the mRNA expression levels in regenerated transgenic plant lines overexpressing a farnesyl diphosphate synthase (FPS), a sesquiterpene (patchoulol) synthase (PTS), or both.
**Figure 3** shows protein expression levels (western blotting) of the patchoulol synthase (PTS), farnesyl diphosphate synthase (FPS) and fusion protein FPS-PTS proteins in transgenic plants as measured by immunodetection in regenerated transgenic plants.
**Figure 4** shows patchoulol content in leaves of plants transformed with different constructs. The comparison reveals that high yields of patchoulol (> 500 ng/g of fresh leaf FW) were accumulated in plants expressing both, transgenic HMGR and PTS (8PTS). Plants expressing transgenic PTS only (2PTS) typically accumulate less than 500 ng/g patchoulol.
**Figure 5** shows the organisation of a vector ("pBDON") suitable for *A. tumefaciens-*mediated transformation of plants, in which the region between the attp2 and the attp1 sequence (cm ccdB) may in vitro and site-specifically be recombined to harbour a HMGR, a TS and, optionally, a PRT. The vector comprises border regions for transformation of plants, promoters, terminators, and the attp1/2 recombination sequences, as discussed in further detail in the examples.
**Figure 6A and Figure 6B** show helper vectors ("pTMON" and "pTDUAL") comprising, between the attp2 and the attp1 sequence, one or two genes, respectively, to be inserted by site-specific recombination into the vector pBDON of Figure 5. Figure 6A shows the helper vector suitable to insert one gene at a time into the pBDON vector, while Figure 6B shows the helper vector suitable to insert two genes at a time into pBDON. The vectors comprises promotor, terminator, attB1/2 recombination and place-holder sequences "Gene 1" and "Gene 2", as discussed in further detail in the examples.
**Figure 7** shows the organisation of the T-DNA region of different plant transformation vectors. The HPT region provides hygromycin resistance, PTS encodes a sesquiterpene (patchoulol) synthase, LIS encodes a monoterpene (limonene) synthase, FPS and GPS encode prenyl transferases (respectively a farnesyl diphosphate synthase and a gerany diphosphate synthase). The vector further comprises suitable promoter and terminator sequences.
**Figure 8** schematically shows the construction of pBDON vector (Figure 5).
**Figure 9** schematically shows the construction of pTMON helper vector (Figure 6 A).
**Figure 10** **part I and** **Figure 10** **part II** schematically show the construction of pTDUAL helper vector (Figure 6 B).
**Figure 11** schematically shows the construction of (A) limonene synthase (LIS) and (B) limonene synthase + geranyl diphosphate synthase (GPS) expression vectors for over-expression in transgenic plants.

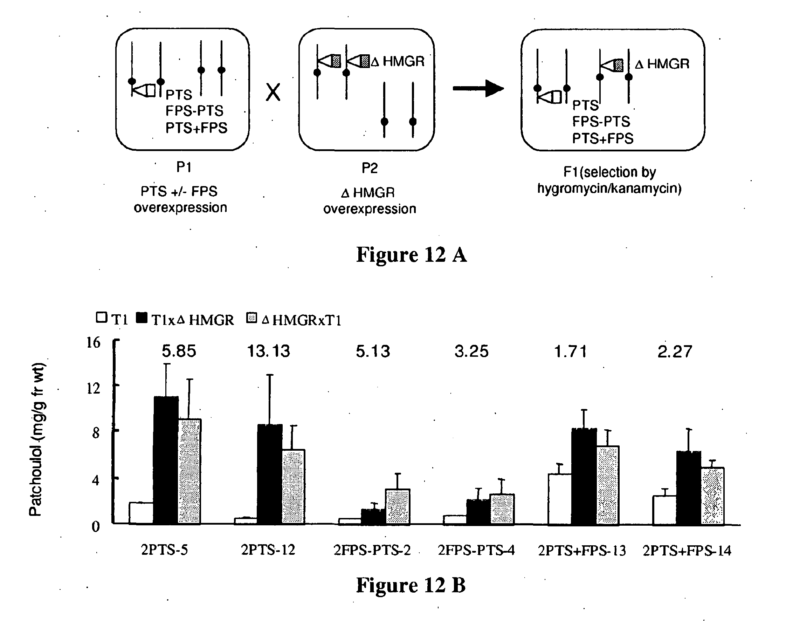

**Figure 12** **A and** **Figure 12** **B** demonstrate the contribution of over-expressing a truncated form of HMGR in combination with over-expressing of PTS and FPS on patchoulol accumulation in transgenic plants. Part A illustrates the genetic crosses performed between plants first engineered with PTS, PTS+FPS or PTS-FPS gene fusions and plants engineered with a truncated HMGR (ΔHMGR) gene. Part R shows the results of the evaluation for patchoulol accumulation of the resulting progeny plants. The patchoulol levels (µg/g fr wt) in parental lines (denoted in white) are shown relative to the corresponding progeny lines (reciprocal crosses - black and grey), with the numbers on top of the graphs indicating the average fold improvement in patchoulol accumulation by addition of the HMGR gene. The ΔHMGR parental line (P2) did not accumulate any patchoulol.
**Figure 13** **A** shows predicted ¹³C-labeling patterns in patchoulol bio-synthesised through the MEP or MVA pathway in seedlings grown on [1-¹³C]-glucose.

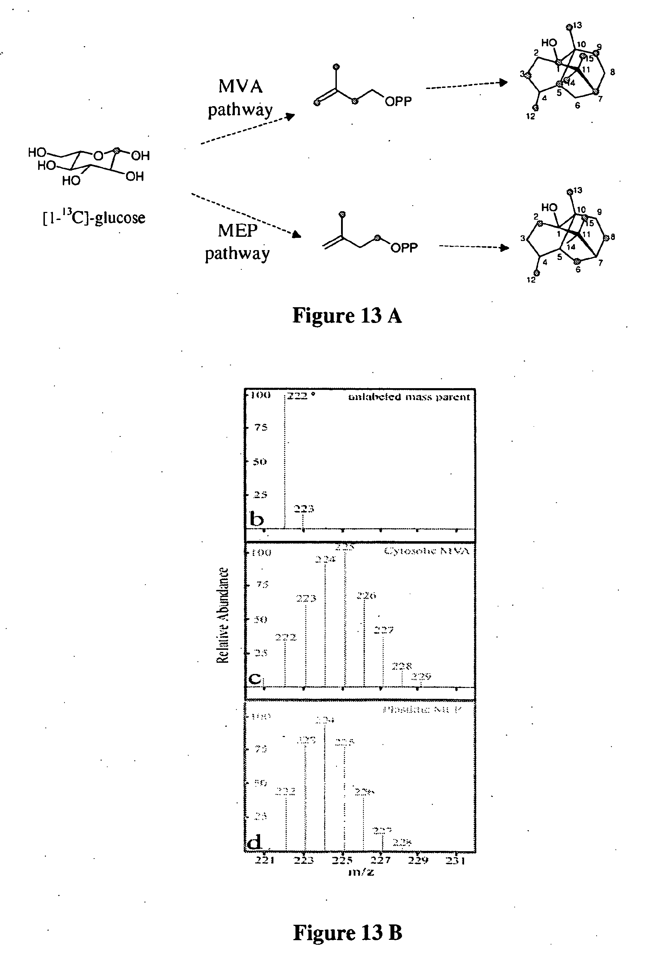

**Figure 13** **B** compares MS parent ions for patchoulol synthesized by plants fed ¹²C-glucose (upper panel), versus synthesis from [1-¹³C]-glucose by plants engineered for cytosolic (middle) or plastidic (bottom) biosynthesis.

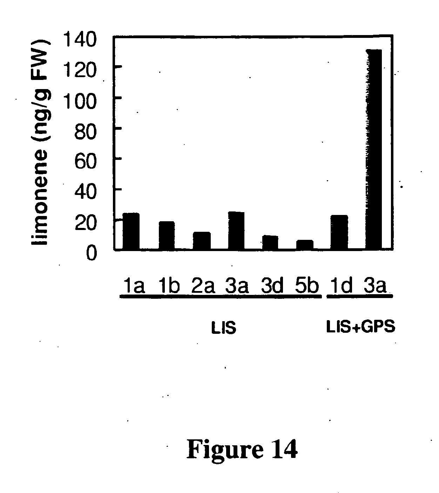

**Figure 14** shows limonene production in plants transformed with transgenes encoding a monoterpene (limonene) synthase (LIS) and, in strains 1d and 3a, with a transgene encoding a geranyl diphosphate synthase in addition.

The sequence listing shows nucleotide sequences used in the preparation of the transformed plants of the present invention. SEQ ID NO: 1 shows the sequence encoding the patchoulol synthase (PTS). Variants of this sequence, for example fused with a nucleotide sequence encoding a His-tag were also used. SEQ ID NO: 2 is a nucleotide sequence encoding an avian farnesyl diphosphate (FPS). SEQ ID NO: 3 shows a nucleotide sequence for the avian FPS fused (bp 1-1134) with a PTS (bp 1144-2802), including a 9bp linker sequence (bp1135-1143) (FPS-PTS). SEQ ID NO: 4 is a nucleotide sequence encoding a limonene synthase from citrus with the plastid targeting signal removed (LIS). SEQ ID NO: 5 is a nucleotide sequence encoding a geranyl diphosphate synthase from *Arabidopsis thaliana* with the plastid targeting signal removed (GPS).

SEQ ID NO: 6-48 are nucleotide sequences of primers used for the present invention.

SEQ ID NO: 49-57 are peptide sequences used to prepare polyclonal antibodies antibodies for PTS (50-54) and FPS (55-48), respectively.

### Detailed Description of Preferred Embodiments

The present invention provides a plant expressing a transgene encoding a HMG-CoA reductase (HMGR), a prenyl transferase (PRT) and a transgene encoding a terpene synthase (TS), these genes being nuclear genes devoid of a plastid targeting sequence.

The plant may be any plant, preferably, it is a plant which is suitable to be transformed according to the present invention. Preferably, the plant is a plant, which naturally produces high amounts of terpenes. For example, the plant is selected from the family of *Solanaceae, Poaceae or Lamiaceae.* For example, the plant is selected from the genera *Nicotiana, Solanum, Sorghum, Arabidopsis, Medicago* (alfalfa), *Gossypium* (cotton), *Brassica* (rape). Preferably, the plant belongs to the species of *Nicotiana tabacum.*

The term "expressing", "expression" and the like, for example "expressing a gene" refers to the fact that a gene is transcribed to mRNA and that proteins encoded by the expressed gene are found in the plant. The term "expressing" also encompasses "over-expression", the latter referring to levels of mRNA, protein and/or enzyme activity over and above what is measured in non-transgenic plants.

The term "transgene" refers to a nucleotide sequence comprising a gene isolated from a foreign organism, generally another kind or species than the plant comprising the transgene.

The term "gene", as present in the word "transgene", for example, refers to a nucleotide sequence that can be translated by the plant of the invention to a protein having the activity indicated, for example a terpene synthase. For the purpose of the present invention, the term "gene" may also include non-coding regions, as long as the final protein construct expressed in the plant shows the activity mentioned.

Accordingly, a gene encoding an enzyme, such as a HMGR, for example, including transgenes, refers to a nucleotide sequence comprising information for the preparation of at least one polypeptide.

The present invention further provides a plant transformed to comprise additional genes, said genes encoding at least a HMGR, a PRT and a TS.

The term "transformed", such as a transformed plant, refers to the fact that a plant has been subjected to genetic engineering. A "transformed plant", includes asexually (vegetative) and sexually derived progeny of an individual plant that has been transformed. For example, a plant obtained by crossing of a plant having individually been transformed with untransformed plants is encompassed by the present invention, if the progeny contains the additional genes and/or transgenes as required by the present invention.

Within the context of this specification the word "comprises" is taken to mean "includes, among other things". It is not intended to be construed as "consists only of".

The term a plant transformed to comprise "additional genes" refers to plants that, due to genetic engineering, comprise new or foreign DNA (for example, genes) inserted into any position of the entire genome of a plant (nuclear, mitochondrial and plastid) that were not present at this position before the genetic engineering.

The plants of the present invention comprise genes encoding an HMG-CoA reductase (HMGR). A HMGR is an enzyme that is capable of catalizing the reductive deacylation of 3-hydroxy-3-methylglutaryl-Coenzyme A to mevalonate. The term HMGR encompasses all classes of HMGR, in particular classes I and II. The term HMRG encompasses enzymes having EC number EC 1.1.1.34. HMGRs are present in all eucaryotic organisms and also in many bacteria.

The present invention is not limited to a specific nucleotide sequences encoding a specific HMGR, much to the contrary, any HMGR may be selected for the purpose of the present invention. The skilled person may thus select any gene isolated from any organism encoding a HMGR from the literature or from public databases. In addition, the skilled person may routinely isolate new genes encoding HMGRs, for example, from any organism. Given that over 60 nucleotide sequences encoding HMGRs from various organisms have already been isolated and are readily available to the skilled person, a list of specific HMGRs need not be provided here.

Preferably, the HMGR gene is heterologuous to the plant of the present invention. More preferably, it is a non-plant HMGR, for example a HMGR gene isolated from an animal is used.

Preferably, the gene encodes a soluble and not membrane-bound HMGR. This may be achieved by truncating the membrane-binding region encoded by a nucleotide sequence and using a nucleotide sequence encoding a HMGR devoid of a membrane binding region. Alternatively, genes encoding an HMGR lacking a membrane domain have been isolated from *Leishmania major* and may also be used for the purpose of the present invention.

Preferably, the gene encoding a HMGR is under the direction of a strong, unregulated constitutive promoter.

Tobacco plants comprising additional genes encoding a HMGR and/or over-expressing HMGR are disclosed in US 5,306,862, US 5,349,126, US 5365,017, US 5,589,619, for example. These plants may be used for the purpose of the present invention, and may be transformed to comprise at least a transgcnc and/or a copy of an additional gene encoding a TS.

The plants of the present invention comprise at least one gene encoding a TS. TSs are enzymes that catalyse the formation of a terpene from a given precursor compound. According to a preferred embodiment, the TS is a mono- and/or a sesquiterpene synthase. A monterpene synthase is an enzyme that catalyses the formation of a monoterpene (C₁₀-compound) with geranyl diphosphate as precursor. A sesquiterpene synthase is an enzyme capable of catalizing the formation of a sesquiterpene (C₁₅) from farnesyl-diphosphate.

Therefore, in contrast to HMGRs, which always produce mevalonate, the TS of the present invention may be selected from different enzymes having different reaction products. Accordingly, the term TS refers to many different TSs capable of synthesising, altogether, thousands of different terpene compounds.

An important advantage of the present invention is thus the large choice of selecting any desired TS. Depending on the terpene that the skilled person wants to produce or have accumulated in the plant of the present invention, the nucleotide sequence that is capable of synthesising the respective terpene may freely be selected. In this way, the present invention provides a cost-efficient platform for the production of any terpene of interest.

For illustrating the principle of the present invention, a few TS for which the genetic information is available are provided as examples: Examples of monoterpene synthases are the limonene synthase (LS) (Ohara *et al,* 2003) and S-linalool synthase (LIS). (Lucker *et al* 2001).

According to a preferred embodiment, the TS is a sesquiterpene synthase. Given that thousands of sesquiterpenes have been described so far, a corresponding number of sesquiterpene synthases may serve as a pool from which the gene encoding a sesquiterpene synthase may be selected from. Accordingly, the gene encoding a sesquiterpene synthase may be selected from publicly accessible databases, from the literature, or from so far undiscovered genes that the skilled person can readily isolate from organisms by routine experiments and well-known isolation procedures. For example, the TS may be capable of synthesizing β-caryophyllene, α-humulene, germacrene A, B, C, D, valencene, aristolochene, vetispiradiene, patchoulol, cubebol, γ-curcumene, (-)-germacrene D, (+)-germacrene D, bicyclo-germacrene, and/or δ-cadiene synthase. The genes of some of the TSs capable of synthesising the above sesquiterpenes are disclosed in the international applications WO 2005/052163 and WO 2004/031376.

For example, the terpene that can be synthesized by the TS is a fragrance, flavor, medicament, insecticide, fungicide, and/or herbicide. Preferably, it is a fragrance.

A "terpene" is a hydrocarbon based on or composed of isoprene units (C₅H₈), which may be cyclic or acyclic. For the purpose of the present invention the terms "terpene", "mono-", "di" and/or "sesquiterpene" also include terpene derivatives, such as terpenoids, which include hydrocarbons that have undergone one or more steps of functionalization such as hydroxylations, isomerizations, oxido-reductions, dimethylation, or-acylation, for example.

The genes encoding a HMGR and a TS may, for example, be present in the form of one single gene encoding a fusion protein having both, TS and HMGR activity.

According to a preferred embodiment, the plant of the present invention further expresses a transgene encoding a prenyl transferase (PRT), or is transformed to comprise additional genes encoding a PRT.

Prenyl transferases (PRT), also called polyprenyl diphosphate synthases or polyprenyl pyrophosphate synthases are enzymes that catalyse alkylation steps involving dimethylallyl diphosphate (DMAPP) and one or more IPP residues, for example farnesyl diphosphate (FPP), geranyl diphosphate (GPP), or others. The term PRT also includes one or several distinct enzymes capable of catalysing one or a sequence of reactions leading to the polyprenyl diphosphate precursor for the various terpenoid families. Therefore, the at least one nucleotide sequence encoding a PRT, for the purpose of the present invention, encompasses sequences encoding for polypeptides having quaternary structures comprising homo- and hetero mixtures of mono-, di-, tri-, terra-, and ologomeric proteins. In particular, the PRT may be a monomer, a hetero- and/or a homo-dimer. The geranyl diphosphate synthase from oil glands of peppermint (*Mentha piperita*) may serve as an example for the complex genetic organisation of certain PRTs encompassed by the present invention, as this enzyme has been purified and was revealed to be a heterodimer, with both subunits required to produce GPP from IPP and DMAPP precursors.

Preferably, PRTs encompass enzymes classified under EC number EC 2.5.1, for example.

In a preferred embodiment, the at least one gene encoding a PRT is a geranyl-diphosphate (GPP) synthase and/or farnesyl-diphosphate (FPP). GPP and FPP are the precursors for mono- and sesquiterpenes, respectively.

Geranyl-diphosphate synthases (GPS), also called dimethyl-allyl-transtransferases, are examples for PRTs.

According to a preferred embodiment, the PRT is a farnesyl diphosphate synthase (FPS). FPSs are enzymes that catalyse, for example, the condensations of geranyl diphosphate (GPP) with IPP to give farnesyl diphosphate. Preferably, the FPS is capable of catalysing the sequential condensation of isopentenyl diphosphate (IPP) with the allylic diphosphates, dimethylallyl diphosphate (DMAPP), and then with the resultant geranyl diphosphate (GPP) to the ultimate product farnesyl diphosphate.

Preferably, the gene PRT is selected to provide the precursor for the selected TS in the plant of the present invention. For example, if the plant comprises an additional gene encoding a sesquiterpene synthase it further comprises an additional gene encoding a farnesyl-diphosphate synthase.

Similar to the genes encoding HMGRs and TSs, genes encoding PRTs are publicly available and may be selected by the skilled person from various origins. Publicly available databases suitable for obtaining nucleotide sequences encoding HMGRs, PRTs and/or TS are, for example, the database of the European Bioinformatics Institute, (http://www.ebi.ac.uk/swissprot/index.html), the EXPASY database (http://www.expasy.org/enzyme/), the NCBI database (http://www.ncbi.nlm.nih.gov) and many others. For the mere purpose of illustrating the many possibilities of FPSs available to the skilled person that could be used for the purpose of the present invention, one could cite a geranyl diphosphate synthase isolated from *Ips pini* (NCBI Accession number (AN): AY 953508.1), a farnesyl diphosphate synthase isolated from *Vibrio fischeri* (NCBI AN: YP 203660), and an avian farnesyl diphosphate synthase reported by Tarshis et al (1994). Of course, any other PRT could be selected from any database or origin.

According to a preferred embodiment, the products of the tansgenes and/or additional genes are directed to the cytosol of cells of the plant. Gene product may be directed to the cytosol of the plant by transforming plants to comprise copies of the genes in the nuclear chromosome, said genes being free from any plastid targeting sequence, and/or any other organelle targeting information. This is relevant, for example, for many of the known monoterpene synthases, which often comprise plastid-targeting sequences, their principle site of activity being the plastid. In this case, the plastid targeting sequence should be removed from the additional gene and/or transgene encoding a TS and/or a PRT, before transforming a plant with it. Accordingly, in a preferred embodiment, the genes and/or transgenes are expressed in nuclei of the plant. Preferably, they are part of a plant chromosome.

Preferably, the gene products (TS, HMGR, and PRT) encoded by the additional genes and/or transgenes present in the plants of the invention are active. Preferably, they are active *in vivo* and/or *in vitro.* Standard assays may be used to determine activity of enzymes in transformed plants. An essay for quantitatively assessing HMGR activity in transformed plants is disclosed in US 5,349,126, Example 2, referring to the method of Chappell et al, Plant Physiol., 85:469-473 (1987). In WO 04/031376, Example 4, an enzyme function assay for a sesquiterpene synthase is disclosed. Assays for assessing PRT activity may be found in the literature, too.

In a preferred embodiment, the plant of the present invention accumulates, if compared to a native, untransformed plant, at least 1.2 times of a terpene that can be synthesised by the TS encoded by the transgene and/or additional gene. Preferably, the transformed plant accumulates at least 1.5 times, twice, three times, more preferably at least four times and most preferably at least 6 times as much of the specific terpene.

According to another embodiment of the invention, the transformed plant accumulates at least 400 ng / per g of fresh leaf of a terpene that can be synthesized by the TS encoded by the transgene and/or additional gene. Preferably, the transformed plant accumulates at least 500 ng, more preferably at least 800 ng, even more preferably at least 1000 ng, still more preferably at least 2000 ng, even more preferably at least 5000 ng, still more preferably 7000 ng and yet more preferably at least 8 µg of the specific terpene that can he synthesized by the recombinant TS present in the transformed plant. Most preferably, the transformed plant accumulates at least 10000 ng or even more of the specific terpene per g of fresh leaf. For example, the transformed plant accumulates 2 - 16 , preferably 4 - 12 µg of terpene per g of fresh leaf. The quantity of the specific terpene may also be expressed in weight of dry matter. In this case, dry matter plant materials, and in particular leaves, corresponds to about 10% of the values for fresh weight. Accordingly, at least 4, 5, 8, 10, 20, 50, 70 or 100 µg terpene per g of dry leaf are accumulated.

In case the native, untransformed plant already produces the specific terpene that can be synthesised by the TS, the values valid for the transformed plant are added to the amount of the specific terpene natively present in the untransformed plant.

For determination of the content of the specific terpene in the plant of the present invention, any plant organ producing or accumulating terpenes may be taken as a reference. Preferably, green leaves having the same age (preferably adult leaves) are taken from the transformed and non-transformed plant, respectively, for comparison. The analysis is preferably conducted according to the protocol "Terpene analysis" outlined in the examples. Preferably, fresh leaves are analysed directly after cutting from the plant. Leaves may be frozen after harvesting and be analysed in the frozen state.

According to a preferred embodiment, the transformed plant according to the invention is the transformed *Nicotiana tabacum cv Xanthi* deposited at the ATCC under the sample designation "8hsPTS-10" and the ATCC number ATCC PTA-6706.

Preferably, the transformed plants may comprise knock-out, down-regulating, deletion or other forms of deleterious mutations in selected wild-type genes participating to the terpene biosynthetic pathways suitable to deviate the carbon flux away from conventional terpenes to that of the TS of the present invention. Preferably, these conventional, other terpene pathways are down-regulated. For example, the plants of the present invention may have one or more non-, or reduced functional genes downstream the FPP, GPP and/or GGPP synthases encoding genes leading to the synthesis of sterols, polyprenoids, phytols and carotenoids. In this way, carbon-flux may more efficiently be directed towards the synthesis of the specific terpene.

The plants of the present invention can be transformed with vectors comprising a nucleotide sequence encoding at least one PRT and/or at least one TS. Preferably, the vector further comprises a nucleotide sequence comprising a HMGR-encoding gene. Preferably, the vector comprising a nucleotide sequence encoding one, two or all selected from the group consisting of HMGR, TS and/or PRT is free of a plastid targeting sequence linked to the HMGR, TS, and/or PRT.

Preferably, the vector further comprises promoter and terminator sequences, preferably up-stream and downstream the nucleotide sequence including the plastid targeting sequence and the gene for the HMGR, TS and/or PRT, respectively. Preferably, the vector comprises strong constitutive promoters. Commercially available examples for promoters are the FMV, figwort mosaic virus, ACT2, actin, MAS, mannopine synthase and the NOS promoter. Preferably, the promoters are selected to be independent from control and/or feed back regulation mechanisms of the untransformed host plant. Preferably, the recombinant HMGR and TS have up-stream promoter sequences. More preferably, they have each different promoter sequences.

Optionally, the vector may further comprise tags suitable for antibody binding, such as His-tags and/or myc tags.

Preferably, the vector further comprises a marker suitable for selecting transformed plants. For example, the vector may comprise genes conferring a hygromycin or a kanamycin resistance, or any other kind of marker suitable to select for successfully transformed plants.

Preferably, the vector further comprises left and right T-DNA border regions, flanking the HMGR, TS, and PRT, including optional promoter, terminator, and/or tagging sequences, as well as plant resistance markers, in order to facilitate transfer and integration of the structural genes into the plant nuclear genome.

Accordingly, the vector preferably comprises structural genes encoding a HMGR, PRT and/or a TS, and a marker for selection of transformed plants, flanked by left and right border regions facilitating insertion into the plant genome.

Preferably, the vector further comprises, outside the left and right border region, a marker for selecting positive bacterial transformants used to clone bacteria comprising the vector or used for transformation of plants (*A. tumefaciens*).

In a preferred embodiment, the vector is the plasmid deposited at the ATCC with the sample reference "pBhsPTS" and the ATCC number PTA-6707.

In an aspect, the present invention relates to a method for preparing a transformed plant, the method comprising the steps of
- transforming plant material to comprise additional genes, said additional genes encoding a HMGR and a TS and being nuclear genes devoid of a plastid targeting sequence, and,
- regenerating transformed plants from said plant material.

For the step of transforming plant material, any method for transforming plants with the genes of the present invention may be employed. For example, plant cells that have been stripped of their protective cell walls take up pure DNA when treated with certain membrane-active, agents or with electroporation. DNA can also be microinjected into target plant cells using very thin glass needles. A recently developed method of plant transformation, biolistics, involves accelerating very small particles of tungsten or gold coated with DNA into cells using an electrostatic pulse, air pressure, or gunpowder percussion.

The present inventors have obtained good results with the *Agrobacterium tumefaciens-mediated* transformation. Preferably, plasmid constructs (vectors) comprising at least one of the structural genes of the present invention, flanked by nucleotide sequences that allow integration of the structural genes into the plant nuclear chromosome DNA are used, such as those disclosed above.

Accordingly, in an embodiment of the plants, the methods.or uses of the present invention, the genes encoding HMGR, the TS, and the PRT, are nuclear genes present in the nuclear chromosome of the plant of the present invention.

Transformation of plant material with DNA constructs, for example comprising genes encoding a HMGR, TS, and/or PRT may be performed by standard methods, see Schardl et al, Gene (1987) 6: 61:1-11; Berger et al, Proc Natl Acad Sci USA (1989) 86:8402-8406; and Horsch et al, Science (1985) 27:1229-1231, the latter method describing the "leaf disk method", which is particularly suitable for transforming *Nicotiana tabacum.*

The methods of the present invention further comprise the step of regenerating the transformed plant from the transformed plant material. Methods for regenerating entire plants from transformed plant material are routinely applied by the skilled person. For example, the method disclosed by Horsch *et al* (1985) may be employed. In general, segments of leaves inoculated by insert DNA containing *A. tumefaciens* may be grown in selective media until callus and regenerated plant shoots are evident. Shoots of 1-3 cm in size may be transferred to T-Media (Schardl, 1987) containing antibiotics to stimulate root development. Once root systems are established, the plantlets may be transferred to commercially available potting soil and propagated in a greenhouse. Accordingly, in a preferred embodiment, the method for preparing a transformed plant further comprises the step of cultivating the transformed plant.

The method of the present invention for preparing transformed plants has the purpose of obtaining accumulation of a desired terpene in the transformed plant. The transformed plants are thus preferably screened for transformants accumulating high amounts of the terpene in the plant. The steps of the methods of preparing transformed plants are thus equally suitable as methods for altering the content of a terpene in a plant, which latter methods represent further preferred embodiments of the present invention. Similarly, the above disclosed methods and plants also illustrate an aspect of the present invention, which is the use of nuclear genes devoid of a plastid targeting sequence and encoding a HMGR, a TS and a PRT for producing plants having an altered terpene content, and preferably an increased content of a specific terpene.

The present invention further relates to a method for producing a terpene, the method comprising the step of isolating the terpene from the plant of the invention or from the plant obtainable by the methods of preparing transformed plants. The transformed plant may be cultivated and harvested, preferably in sufficiently high yield and/or on a sufficiently large scale to render the process economically favourable. The specific terpenes of the invention may be isolated by any method used in the art including organic solvent extraction or distillation and may be quantified and/or identified by gas chromatography (GC)-mass spectrometry (MS).

### Examples

The following examples are intended to illustrate the invention without limiting the scope as a result. Methods and protocols of the examples are generally performed following standard protocols supplied by the manufacturer of specific materials or kits, or by following well-established protocols defined by Sambrook et al (1989) and Ausubel et al. (1987).

All primers used are listed in the attached sequence listing. For some method or protocol steps, reference is made to the literature, which is listed further below in more detail.

### Example 1: Selection of Plant material

The homozygous line of *Nicotiana tobacum* 'Xanthi' line containing a truncated hamster hydroxymethyl-glutaryl-CoA reductase gene, referred to hereafter as 14-8, and a F2 segregating sibling line lacking the transgene, hereafter referred to as 14-2, were developed as described by Chappell et al. (1995) and in patents US 5306862, US 5349126, US 5365017. In brief, the hamster gene first characterized by Chin et al. (1984) was modified by removing nucleotides 28 to 1023 to generate a truncated form of the gene referred to as delta-227 HMGR. A BamH1/Sst1 fragment of the truncated HMGR cDNA was isolated and inserted into an intermediate plasmid vector, pKYLX 61, and an EcoR1/Cla1 fragment of that construct was subsequently inserted into the corresponding site of pKYLX71 to generate the pKYLX71-truncated HMGR gene construct. The Ti-plasmid series pKYLX61 and 71 were developed by Schardl et al. (1987).

The pKYLX71-HMGR plasmid was mobilized into *Agrobacterium tumefaciens* harboring the disarmed Ti plasmid GV3850 using standard triparental mating procedures (Schardl et al., 1987). Leaf discs of *Nicotiana tabacum* L. cv. Xanthi were used as plant material and were inoculated with the *Agrobacterium* carrying the pKYLX71-HMGR construct and transgenic plants were regenerated using the standard transformation and regeneration protocols described in Schardl et al. (1987) and Horsch et al. (1985).

Primary transformed and regenerated plants (R0 generation) were grown and allowed to self-pollinate and the subsequent seed collected (R1 generation). R1 seeds were grown as individual plants under standard greenhouse conditions, allowed to self-pollinate and the R2 seed generation collected. The R2 seed generation was subsequently screened for the presence of the HMGR transgene.

Screens for the presence of the HMGR transgene constructs were initially based on the presence of the kanamycin resistance marker which was engineered into the plants as part of the pKYLX71-HMGR construct (Chappell et al., 1995). Seeds plated onto germination media containing 100 mg of kanamycin/L were scored for germinated seedlings exhibiting photobleaching (all white seedlings, those lacking the kanamycin resistance marker, and thus homozygous lines lacking the transgene constructs) versus all green seedlings (green seedlings harboring the kanamycin resistance marker, and thus homozygous lines containing the transgene constructs). Seed from these identified lines were subsequently propagated in the greenhouse (without kanamycin selection) and screened for the presence of the engineered HMGR construct by chemical analysis for sterols (Chappell et al., 1995). Line 14-8 exhibited sterol levels 2-10 fold higher than control plants and all seedlings grew normally in the presence of kanamycin, while line 14-2 contained sterol levels comparable to non-transformed, control plants and seedlings germinated in the presence of kanamycin were all photobleached white.

Plant lines 14-8 (IIMGR-over-expressing) and 14-2 (control) were used in all subsequent transformation work.

### Examples 2 - 5. Construction of Recombination and Plant Transformation Vectors

The hygromycin selection marker (Hajdukiewicz et al., 1994) was chosen for creating a selection marker for transformed plants. New vectors were engineered with appropriate recombination cloning sites as described by Hartley et al. (2000).

### Example 2: Development of the pBDON vector

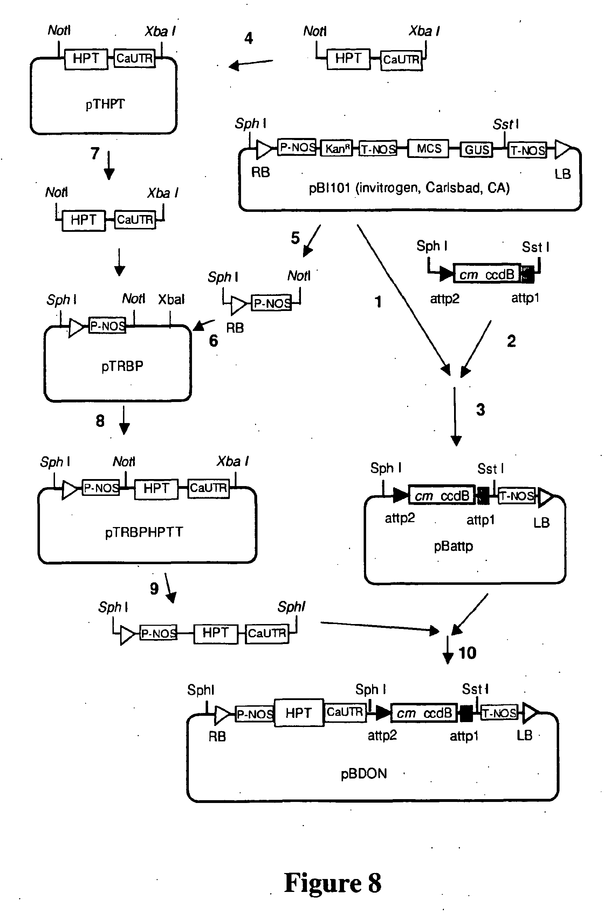

The pBI101 vector (Invitrogen, Carlsbad, CA) was digested with the restriction enzymes Sph1 and Sst1 and the DNA fragment corresponding to the plasmid vector (not including the RB border and NPTII gene cassette) was isolated by agarose gel purification (Sambrook et al, 1989), (1) in Figure 8. In parallel, an attp recombination cassette including the ccdb gene and chloroamphenical resistance gene was amplified from the pDON221 vector (Invitrogen, Carlsbad, CA) using standard PCR conditions with primers Attp1-SstI-FW and Attp2-SphI-RV (2). The PCR amplified DNA fragment was restricted with the Sph1/Sst1 enzymes, gel purified and ligated into the corresponding sites of the similarly digested pBI101 vector described above to yield the intermediate pBattp vector (3).

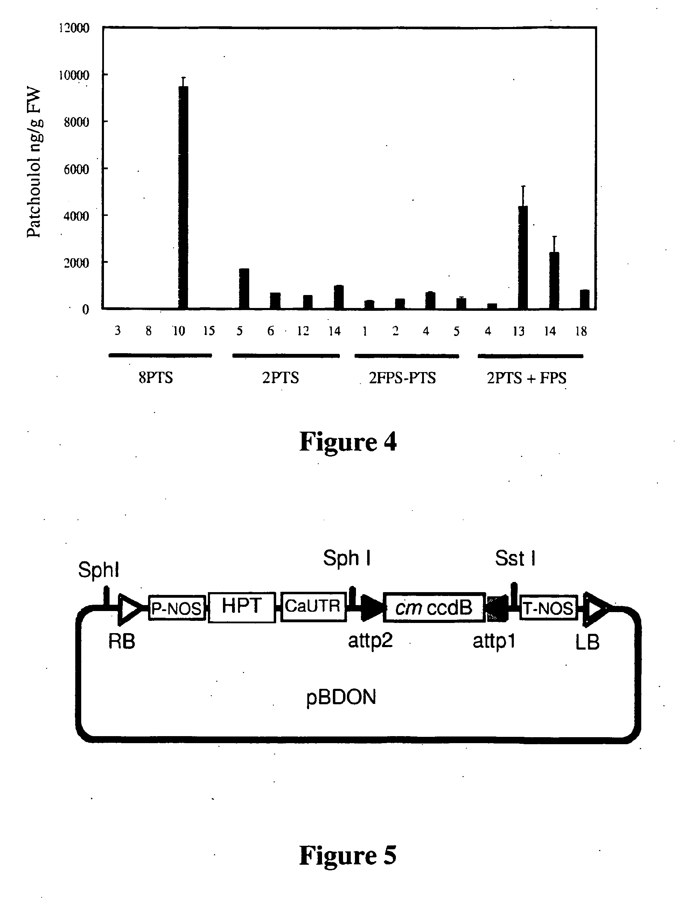

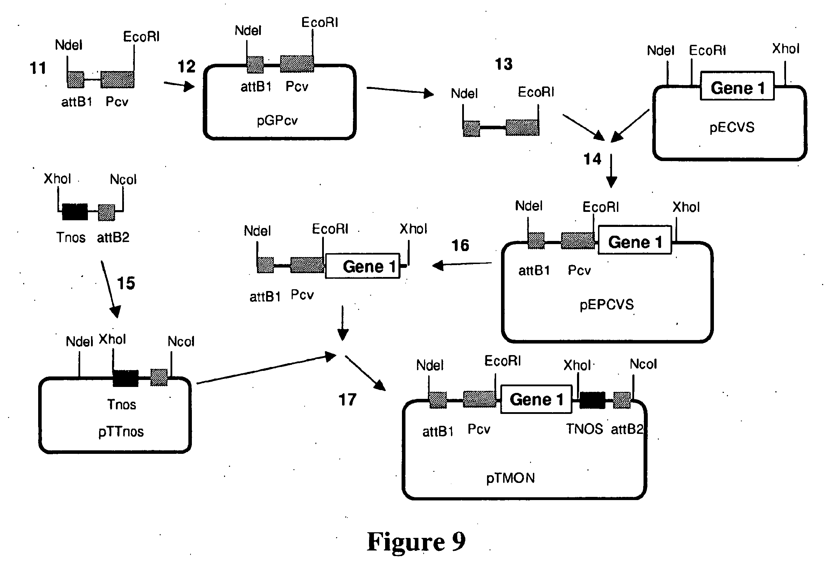

A hygromycin gene cassette was prepared in a 2-step process. First, the hygromycin gene (HPT) and CaUTR (termination sequence) was PCR amplified from the pCAMBIA1301 (Cambia, Canberra, AU) vector using the PCR primers HPT-NotI-FD and HPT-Xba1-RV, and T/A cloned (Taq-Amplified PCR products directly from the PCR reaction mix) into the pT7Blue vector (Novagen, Madison, WI) to yield pTHPT (**4**). The right border (RB) and the NOS-promoter (P-NOS) regions were amplified from pBI101 using the PCR primers TB-SphI- FD and TB-NotI-RV (**5**), then T/A cloned into the pT7Blue vector giving rise to vector pTRBP (**6**). The hygromycin resistance gene cassette was released from vector pTHPT via digestion with Not1/Xba1 (**7**), and cloned into the similarly digested pTRBP vector, resulting in vector pTRBPHPTT (**8**). The NOS promoter-hygromycin-CaUTR cassette was then amplified from this vector using the primers TB-SphI- FD and HPT-SphI-R (**9**). The amplified product was digested with Sph1 and ligated into the corresponding site of pBattp, yielding the pBDON vector (**10**).
The pBDON Ti-vector (Figures 5, 9) contains an NPTII selection marker outside the T-DNA region for selection in bacteria and the hygromycin gene (HPT) for plant transformation selection. The embedded attp cassette hence provides for the easy insertion of target gene constructs flanked with attB sites into the pBDON vector.

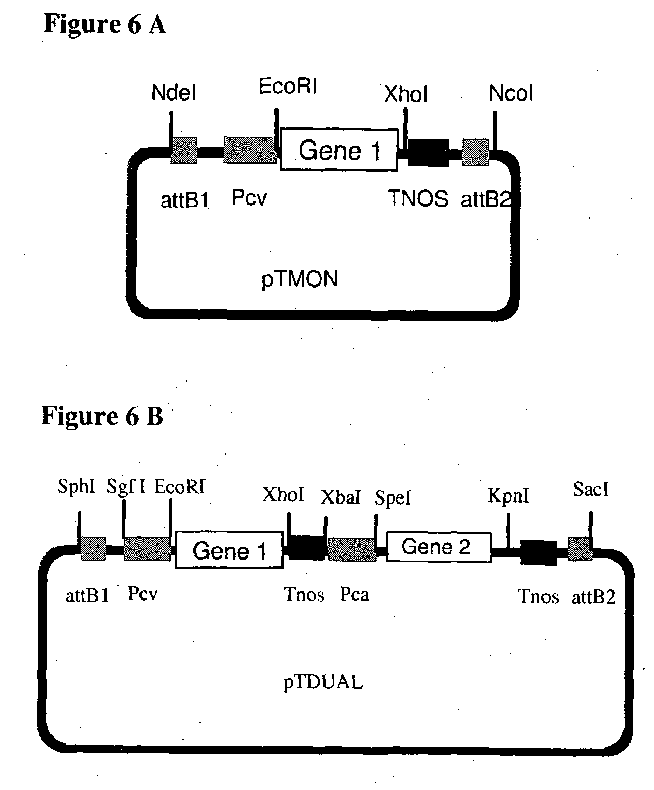

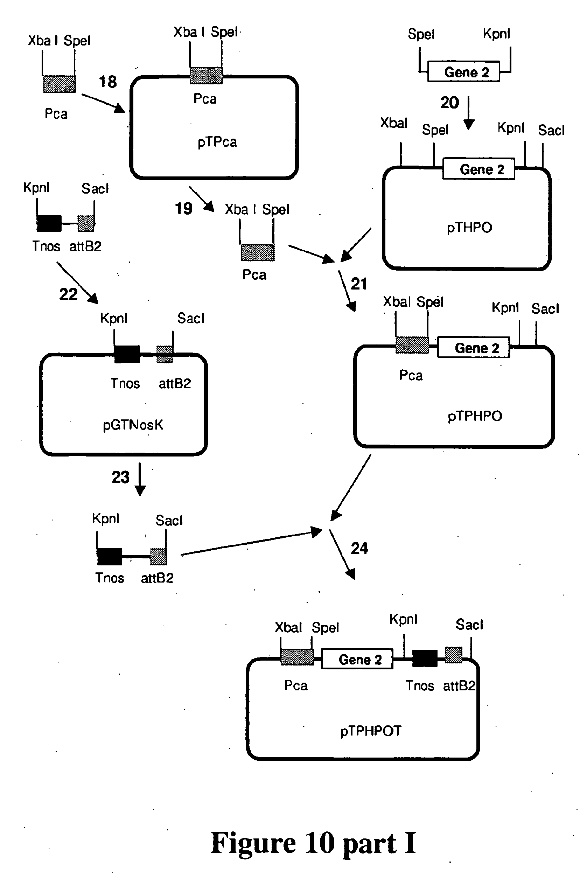

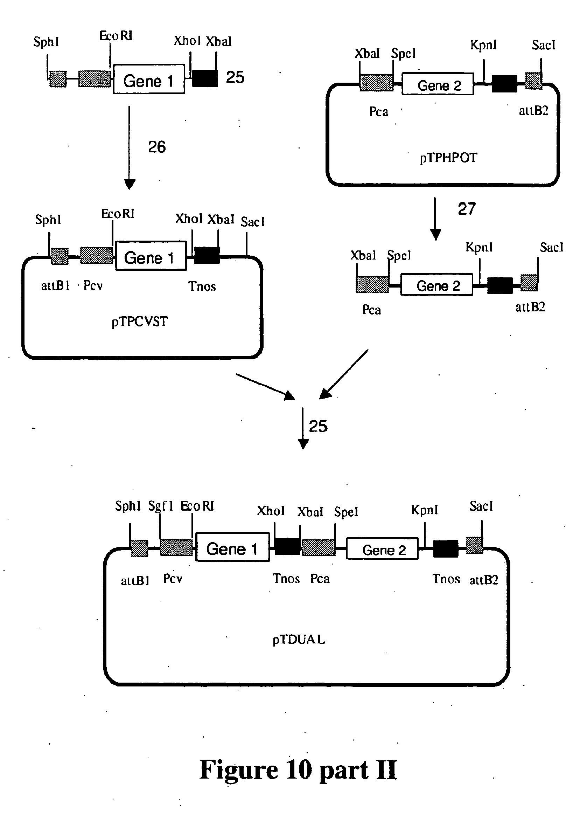

Development of helper vectors with attB sites:
Two different attB help vectors were constructed following the protocol given in Figures 9 and 10. One for single gene insertion (pTMON, Figure 6 A) and the other for two target gene insertions into the pBDON vector (pTDUAL, Figure 6 B).

### Example 3: Generation of the pTMON vector (Figure 9)

The pTMON vector was constructed by first amplifying the cassava mosaic virus (Pcv) promoter from a modified pBI101 vector with a forward PCR primer containing a Nde1 restriction site and an attB1 sequence embedded into the primer CSMV-ATTB1-SGFI-FD, and the reverse primer CSMV-ECORI-RV containing an EcoR1 site (11). The PCR fragment was T/A cloned into the pGem-Teasy vector (Promega, Madison, WI) (12), then re-isolated as a Nde1/EcoR1 digestion product. The isolated digestion product (13) was ligated into the corresponding restriction sites of the pECVS vector, a pET28a derivative harboring an optional placeholder gene, "Gene 1", NCBI accession number CQ813508) to generate pEPCVS (14).

In parallel, the NOS terminator (TNOS) sequence of pBI101 was amplified with the forward primer Tnos-XhoI-FW and the reverse primer Tnos-attB2-RV, which incorporated an attB2 recombination site downstream of the TNOS sequence. The PCR fragment was T/A cloned into the pGEM-Teasy vector, yielding the pTTNOS vector (15). An Nde1/Xho1 digestion fragment of pEPCVS (16) was subsequently ligated into the corresponding sites of pTTNOS to generate pTMON (17) (Figures 6A, 9).

The pTMON vector was constructed for insertion of a single target gene behind a cassava mosiac virus promoter (Pcv) (Frey et al. 2001) and followed by the Nos terminator sequence.

In some cases, a His-Tag sequence was engineered into the pTMON vector by digestion with EcoR1, followed by linker/ligation with the primer pair His-EcoF and His-EcoR. Subsequent mutation was designed to abolish the His-Tag open-reading frame sequence, but to maintain convenient Asc1 and EcoR1 restriction sites. This was accomplished using standard PCR reaction conditions with primers mHisPTS-F and mHisPTS-R, and transforming the PCR reaction products into bacteria to recover the mutated pTMON plasmid.

### Example 4: Generation of the pTDUAL vector (Figure 10 I and II)

The pTDUAL vector was constructed in a multi-step process (Figure 10, part 1 and 2). First, the cauliflower mosaic virus promoter (Pca) (Benfy et al. 1990) was amplified from the pBI121 vector (Invitrogen) using the primers PCaMV-XbaI-FW and PCaMV-SpeI-RV, and T/A cloned into the pT7Blue vector (**18**). The promoter element was subsequently released from this vector by digestion with Xba1 and Spe1 (**19**). In parallel, an optional placeholder gene ("Gene 2"), was amplified from a *Hyoscyamus muticus* cDNA library obtained from an elicitor treated cell culture (PCT/US06/02265) with the primers 7120D-SpeI-FW and 7120D-KpnI-RV, followed by digestion with Spe1/Kpn1 and ligation into the corresponding site of a pT7Blue vector and yielding pTHPO (**20**). pTHPO was then digested with Xba1/Spe1 and the CaMV promoter fragment similarly released from pTPCa were ligated together to give pTPHPO (**21**).

In parallel to build pTPHPO, the NOS terminator sequence was amplified from the pGTNOS vector with primers Tnos-KpnI-FW and Tnos-attB2-RV3, T/A cloned into the pGem-Teasy vector (**22**) followed by subsequent re-isolation of the fragment by digestion of the pGTNOSK plasmid with Kpn1 and Sacl (**23**). This fragment was then cloned into the corresponding restriction sites of pTPHPO to yield pTPHPOT (part 1 of Figure 10) (24).

In the final steps of constructing the pTDUAL vector, a fragment (**25**) of the pTMON vector spanning from the attB1 site to the nos-terminator sequence downstream of the inserted terpene synthase gene was amplified using starndard PCR conditions. The amplification product was obtained with primers CsMV-attB1-Sgf I-FD and TNOS-XbaI-RV, which also engineered terminal Sph1 and Xba1 sites onto the fragment. The PCR fragment was digested and ligated into the corresponding Sph1/Xba1 sites of the pT7Blue vector, generating pTPCVST (**26**). Finally, an Xba1 to Sac1 digestion fragment (**27**) from pTPHPOT was ligated into the corresponding sites of pTPCVST to create the pTDUAL vector (**25**), which allows for the insertion of 2 gene sequences downstream of strong, constitutive expression promoters (Figure 10 part 2).

The pTDUAL vector (Figure 6 B) was designed for the insertion of two genes into transgenic plants. Expression of the first gene is directed by the cassava mosaic virus promoter (Frey et al. 2001), while expression of the second gene is directed by a cauliflower mosaic virus promoter (Benfey et al. 1990).

Similar to the modifications made to the pTMON construct described above, in some cases a His-Tag sequence was engineered into the pTDUAL vector by digestion with EcoRl, followed by linker/ligation with the primer pair His-EcoF and His-EcoR. Subsequent mutation was also designed to abolish the His-Tag open-reading frame sequence, but to maintain convenient Asc1 and EcoR1 restriction sites. This was accomplished using standard PCR reaction conditions with primers mHisPTS-F and mHisPTS-R, and transforming the PCR reaction products into bacteria to recover the mutated pTDUAL plasmid.

### Example 5: Construction of the patchoulol synthase (PTS) and patchoulol synthase + farnesyl diphosphate synthase (FPP) over-expression vectors

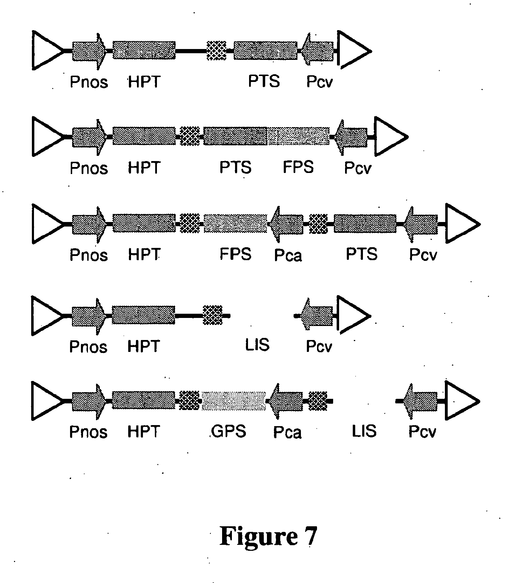

Generation of the PTS and PTS + FPS expression vectors were greatly facilitated by the appropriate recombination cloning sites associated with the pTMON, pTDUAL and pBDON vectors. The PTS (WO 2004/031376) or FPS genes (Tarshis et al, 1994) were amplified with primer pairs PTS-AscF and PTS-XhoR, or FPP-SpeFW and FPP-KpnRV, respectively, digested with either Asc1/Xho1 or Spe1/Kpn1, then ligated into the corresponding sites of the pTMON or pTDUAL, vectors. The FPS-PTS gene fusion was created by amplifying the FPS gene with primers FPS-AscF and FPS-AscR, digesting the resulting PCR fragment with Asc1 and ligating this fragment into the corresponding Asc1 site found 5' to the PTS gene in the pTMON and pTDUAL vectors. The resulting plasmids were then used to mobilize the corresponding PTS, PTS+FPS and FPS-PTS gene cassettes into the pBDON vector by standard recombination cloning (Hartley et al. 2000) generating a family of Ti-plasmid vectors (Figure 7). A plasmid pBDON vector comprising a PTS encoding gene with a His-tag corresponding to the first cassette shown in Figure 7 was deposited under deposit number ATCC PTA-6707.

### Example 6: Plant transformation and regeneration

Individual pBDON vector constructs (Figures 5, 7) were transformed into Agrobacterium tumefaciens strain GV3850 by electroporation (Mersereau et al. 1990) and transformants selected for kanamycin resistance. Selected colonies were verified for the transgene constructs by limited DNA sequencing and subsequently grown in 50 mL of LB media containing 100 mg kanamycin/L. Overnight cultures having an OD600 equal to 0.6-0.8 were concentrated by centrifugation, resuspended in a 30 mL of fresh LB medium (without antibiotic) and used for inoculation of leaf explants as described previous (Chappell et al., 1995; Horsch et al, 1985). In brief, leaves from plants grown under sterile conditions were placed into the Agrobacterium cultures, cut into approximate 1 cm segments, and the leaf segments plated on non-selective media plates (Murashige and Skoog, 1962). After 3 days, the leaf explants were transferred to media plates containing 15 µg/mL hygromycin (Invitrogen, Carlsbad, CA) and 500 µg/mL cefotaxime (Bioworld, Dublin, OH) and subsequently to the same selection media weekly until callus and re-generated plant shoots were evident. Shoots of 1- 3 cm in size were then transferred to T-media (Murashige and Skoog, 1962) (containing the same antibiotics) to stimulate root development. Once root systems were established, the plantlets were transferred to commercially available potting soil and propagated in a greenhouse.

### Example 7: Terpene analysis

Sesquiterpenes extracted from leaf material of transformed plants obtained in Example 6 were identified and quantified by GC-MS analysis. Frozen leaf samples of 200-500 mg were ground in liquid nitrogen, then extracted with 3 mL of a hexane:ethyl acetate mixture (v/v 85:15) containing 200 ng of α-cedrene as an external standard. The extract was partially purified by running the sample over a silica column eluted with the same 85:15 mixture of ethyl acetate: hexane. The eluate was concentrated under a stream of nitrogen to 30 µL before analyzing 1 µL aliquots by GC-MS (Takahashi S, 2005). Samples were injected onto a Trace GC-MS (ThermoFinnigan, Somerset, NJ) equipped with a Rested Rtx-5 capillary column (30 m X 0.32 mm, 0.25 µm phase thickness) operated in the splitless mode with an injector temperature of 250°C and an initial oven temperature of 70°C for 1 min, followed by a 4°C per min gradient to 230°C. Mass spectra were recorded at 70 eV, scanning from 35 to 300 atomic mass units, and compared to library standards (NIST library) and authentic standards for verification.

The estimation of the quantities of patchoulol was based on the ratio of peak areas obtained by total ion monitoring for patchoulol and the internal standard. The response factor of α-cedrene and patchoulol was calculated by co-injection of the same quantities of the corresponding standards and the resulting correction factor was included in the calculation. The same method was used for the quantification of limonene and other terpene molecules.

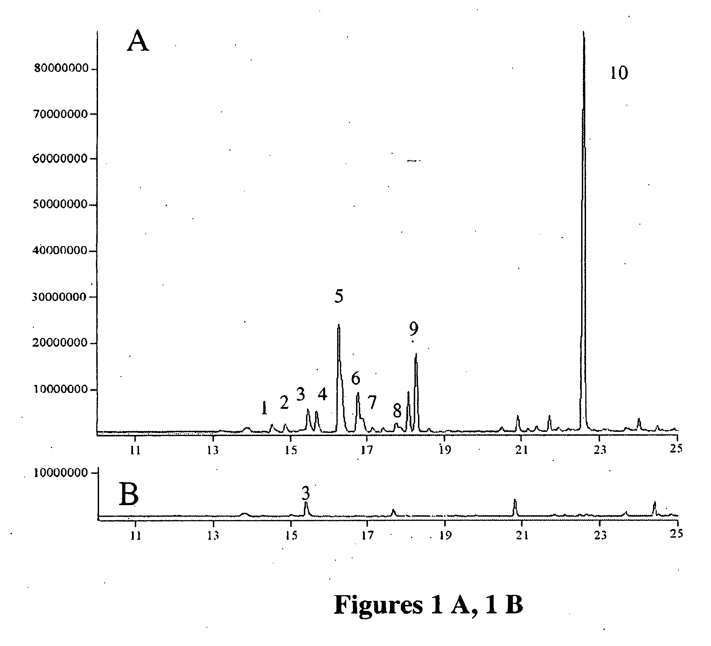

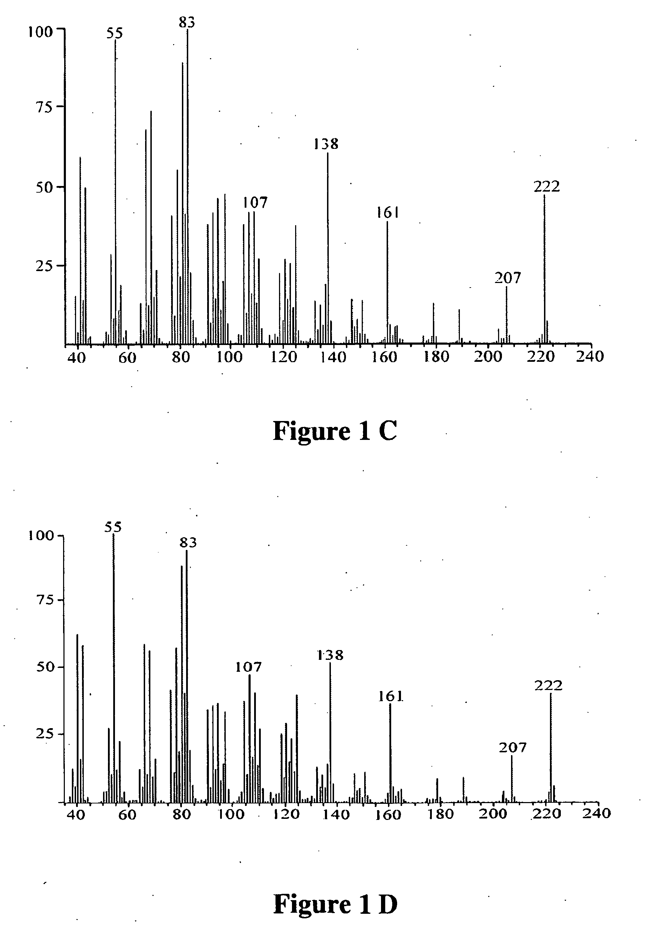

Figure 1 shows the results of terpene analysis of a control tobacco line (WT) and one transformed with HMGR and PTS gene constructs for sesquiterpene content. In Figure 1, a total ion chromatogram for a HMGR over-expressing line transformed with the HisPTS gene constructs (Figure 1A) is compared to that for a control plant (transformed with the HMGR only) (Figure 1 B). Peaks were identified by comparison of their mass spectra to available standards or by spectral matches available in the NIST library. For example, the MS for peak 12 (C) is compared to the MS for authentic patchoulol (D). Other peak identifications: 1-β-patchoulene; 2-β-elemene; 3-α-cedrene (internal standard); 4-caryophyllene; 5-α-guaiene; 6-unknown; 7-α-patchoulene; 8- seychellene; 9-unknown, 10-δ-guaiene; 11-globulol; and 12-patchoulol.

Figure 1 is the result of a GC-MC analysis and illustrates that plants specifically transformed with genes encoding a specific, cytosol-targeted terpene (patchoulol) synthase of interest and a HMGR (A) had an altered terpene content if compared to the wild type (B) and accumulated patchoulol, besides other terpenes synthesised by the same patchoulol synthase.

Figure 4 is a quantitative analysis of sesquiterpene (patchoulol) content in leaves of plants transformed with PTS encoding, recombinant genes, wherein "PTS" alone refers to transformants harbouring a recombinant gene encoding a PTS, "FPS-PTS" refers to a gene encoding FPS fused to a gene encoding a PTS, and "FPS+PTS" refers to separated genes encoding a PTS a FPS. 14-8 + PTS (8PTS) refers to plants over-expressing a HMGR gene according to the invention, while 14-2 + PTS plants (2PTS, 2FPS-PTS, 2PTS + FPS) do not over-express HMGR. Seeds of the plant 8PTS 10 in Figure 4 were deposited under ATCC PTA-6706.

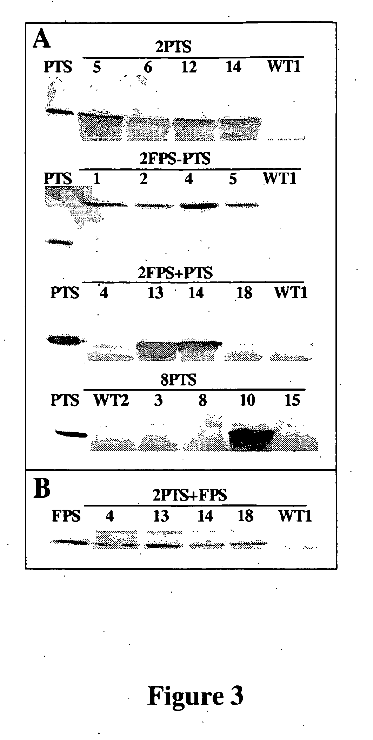

In Figure 4 it can be seen that by transformation of plants with a TS (PTS) encoding gene alone, detectable expression of the protein is observed and was correlated with protein expression levels, but patchoulol accumulation was modest. By co-expressing the TS with a FPS, the accumulation of patchoulol was moderately increased. In contrast, expression of PTS in the 14-8 lines (HMGR over-expression) high patchoulol levels are observed which directly correlate with PTS protein expression levels (strain 10 in Figure 3), compared with the same strain in Fig. 4. This results clearly demonstrating the strong contribution of HMGR to terpene accumulation.

### Example 8: Mendelian Crossing for Obtaining the Plants of the Invention

Because of the difficulty to obtain transformed plants with the 14-8 line, classical mendelian crossing was used to generated plant lines overexpressing PTS alone (PTS), PTS with the FPP synthase (PTS+FPS) or as a fusion protein with the FPP synthase (FPS-PTS) together with HMGR. Transgenic plant lines engineered for expressing either PTS, PTS+FPS or FPS-PTS were initially germinated in the presence of hygromycin (15 mg/L) and only antibiotic resistant plants (comprising the desired construct) were grown up (parent line P1). Parental line P2 is homozygous for a truncated HMGR gene (14-8) (Chappell et al., 1995) and was germinated and grown without selection. Reciprocal crosses (pollen exchange between the two parental lines) yielded F1 progeny seeds that were germinated in the presence of kanamycin (50 mg/L) and hygromycin (15 mg/L) and the resulting resistant plants assessed for patchoulol accumulation as described above in Example 7. These double resistant plants contain the double insertion of a first construct encoding the truncated HMGR and a second construct encoding either PTS, FPS+PTS or FPS-PTS as illustrated in the Figure 12 (part A).

The resulting progeny plants, the F1 generation plants, were then evaluated for there accumulation of patchouol by GC-MS. In part B of Figure 12, the patchoulol levels in parental lines (denoted in white) are shown relative to the corresponding progeny lines (reciprocal crosses - black and grey). The ΔHMGR parental line (P2) did not accumulate any patchoulol. Table 1 bellow shows the numeric values of the same evaluations. The results demonstrate that engineering the patchoulol biosynthesis in the cytosol is significantly enhanced by simultaneously engineering of an unregulated form of HMGR. For all progeny plants resulting from the crosses an increase of 1.5 to 15 fold in patchoulol production was observed.

**Table 1 : Patchoulol Content in Different Transformed Plants and Progeny following Genetic Crossing**

| | Patchoulol (µg/g fresh wt.) | | | |
|---|---|---|---|---|
| Plant line | T1 generation | T1 x HMGR | HMGR x T1 | Fold increase |
| 2PTS-5 | 1.7±0.08 | 10.97±2.96 | 9.02±3.51 | 5.85 |
| 2PTS-12 | 0.56±0.02 | 8.59±4.31 | 6.36±2.08 | 13.13 |
| 2FPS-PTS-2 | 0.42±0.06 | 1.27±0.51 | 3.06±1.31 | 5.13 |
| 2FPS-PTS-4 | 0.72±0.03 | 2.08±1.03 | 2.59±1.29 | 3.25 |
| 2FPS+PTS-13 | 4.40±0.87 | 8.31±1.56 | 6.75±1.42 | 1.71 |
| 2FPS+PTS-14 | 2.43±0.68 | 6.31±1.88 | 4.97±0.55 | 2.27 |

### Example 9: RNA extraction and quantitative RT-PCR analysis

The total RNA of each line was isolated from 500 mg of young leaves with Trizol reagent according to the manufacturer (Invitrogen Life Technologies, Carlsbad, CA). First-strand cDNA was synthesized in 20 µL reactions with 5 µg of total RNA, 200 ng of oligodT12-16 primer and Reverse Transcriptase (Superscript II, Invitrogen Life Technologies) in reaction buffer and conditions as recommended by the manufacturer. One µL of RNase H was subsequently added to the cDNA preparations and incubate at 37°C for 1 hour to remove complementary RNA.

A quantitative RT-PCR method was used to determine mRNA levels in different transgenic lines. Optimal concentrations for linear PCR amplification were determined by varying the amount of added cDNA template and using number of PCR cycles (10, 15, 20, 25 and 30). The primers PTS-8F and PTS-160R were used to amplify PTS gene, while FPP-1F and FPP-160R were used for the FPS gene. Another primer pair, RBCS-FW and RBCS-RV, was used to amplify the RUBisCO small subunit gene as an internal standard. Typical PCR conditions consisted of 1x Taq buffer (as supplied by the manufacturer), 1.5 mM MgCl₂, 0.2 mM dNTP mixture, 0.5 µM of each primers, 1.25 µL cDNA, and 1 unit Taq DNA polymerase in a total of 25 µL mixture. PCR amplifications was carried out for 20 temperature cycles, each consisting of denaturation at 94°C for 30 sec, annealing at 60°C for 30 sec, and extension at 72°C for 60 sec. PCR products were separated by agarose gel electrophoresis, stained with ethidium bromide, and photographed.

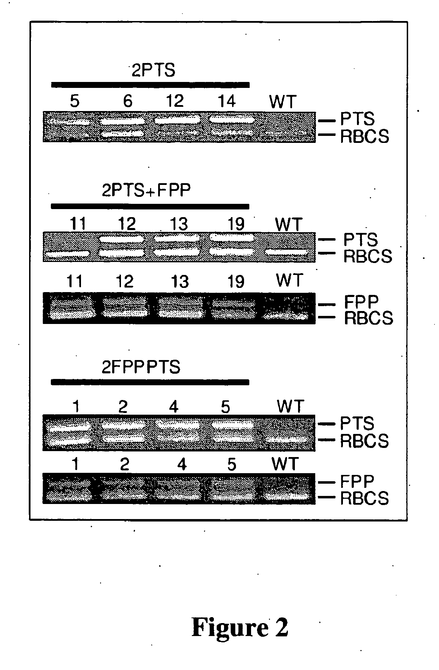

The results of the PTS and FPS mRNA analysis is shown in Figure 2. The figure shows RT-PCR products derived from mRNA isolated from plants transformed with constructs containing a patchoulol synthase (PTS) gene, or a construct containing a patchoulol synthase and a FPS genes (PTS+FPS), or a construct encoding for a fusion protein of FPS and PTS (FPS-PTS). RBCS is the RT-PCR product derived from the Rubisco small subunit mRNA, present in the wild-type (WT) and transgenic plants, and serves as an internal standard.

It can be seen that wild-type (WT) plants did not express PTS or FPS. Most of the lines harboring the PTS, PTS+FPS and PTS-FPS contructs expressed readily detectable levels of the PTS mRNA, but patchoulol accumulation was relatively poorly correlated with the PTS mRNA expression levels.

### Example 10: Western Blotting analysis

A mixture of 4 to 5 synthesized peptides was used as antigens to prepare antibodies of PTS and FPS. The antigenic peptides were predicted by a free software (http://bio.dfci.harvard.edu/Tools/antigenic.html) and selected by direct 3Dstructural analysis (PDB: 1FPS) for FPS or homology model comparison with an available structure EAS (PDB: 5EAS) for PTS. We designed 5 antigenic peptides (PTS46: EELKVEL; PTS108: HATALSFRLLRQHGYRVSCE; PTS353: DEELIKLGAPYRA; PTS462: HVRTAVECYME; PTS475: KVGKQEVVSE) for PTS, and 4 antigenic peptides (FPP41: EFVGFTPQIVR; FPP59: GHPEVGDAVARLKEVLQY; FPP218: YKAIVKYKTAF-YSFYLPVAA; FPP320: PEKVAKVKELYEA) for FPS. All the peptides were synthesized in a 10mg scale at immunological grade by Sigma-Genosys (The Woodland, TX). Each of the peptide mixtures were then conjugated to KLH (Keyhole Limpet Hemocyanin) carrier protein and injected into rabbits by Strategic Biosolutions (Windham, ME). Polyclonal antibodies for PTS and FPS was further purified by preparative antigenic affinity column prepared with CNBR-activated sepharose 4B resin coupled with purified PTS or FPS protein according to the manufacturer's instruction (Amersham Biosciences, Buckinghamshire, England).

To measure PTS and FPS protein levels in transgenic plant material, 100 mg of young leave material was ground in liquid nitrogen, then extracted with 800 µL of 80mM potassium phosphate (pH 7.0), 10% glycerol, 10mM sodium metabisulfide, 15 mM MgCl₂, 10mM sodium ascorbate, 1% polyvinylpyrrolidone, and 14 mM β-mercaptoethanol. The crude protein extracts were centrifuged at 4°C for 20 min at full speed in a table top microfuge, and the resulting supernatant used as the protein source. Samples containing 40 µg of supernatant protein was electrophoresized in a 15% SDS-PAGE gel and blotted into a nitrocellulose membrane (BIO-RAD, Hercules, CA). Membranes were blocked with standard Tween 80/Tris-buffered saline (TTBS) blocking solution containing 5% dried milk before adding the purified PTS or FPS antibody. Membranes were then incubated for 3-7 hrs at room temperature with shaking, washed three times with TTBS, and then incubated with goat peroxidase labeled anti-rabbit IgG (Kirkegaard and Perry Laboratories, Gaithersburg, MD) in TTBS blocking solution for another 3 hours at room temperature. Chemiluminescent detection of the secondary antibody was performed with an ECL reagent kit according to the manufacturer's instructions (Amersham Biosciences, Buckinghamshire, England).

Expression levels of the patchoulol synthase (PTS) and farnesyl diphosphate synthase (FPS) proteins in transgenic plants are shown in Figure 3. The numbers above lanes in Figure 3 represent independent transgenic plant lines engineered to contain a patchoulol synthase (PTS) gene, or a construct containing a patchoulol synthase and a FPS genes (PTS+FPS), or a construct encoding for a fusion protein of FPS and PTS (FPS-PTS). Results are shown for transgenic plants obtained by transformation with terpene synthase gene (patchoulol synthase) alone (2PTS) or with gene encoding a fusion protein (2PTS-FPS) and for plants obtained by transformation with a terpene synthase gene and further comprising a transgenic HMGR gene (8PTS) or further comprising a transgenic FPP synthase (2PTS+FPS). The positive control used are PTS and FPS proteins (respectively lanes PTS and FPS) purified from bacteria transformed to over-express these proteins. Lane WT1, extracts from leaves of a wild-type plant. Lane WT1, extract from leaves of plants only expreesing the HMGR gene. Panel A shows membranes probed with the PTS antibodies and panel B shows a membrane probed with the FPS antibodies.

It can be seen in Figure 3 that no protein was imunologically detected in the wild type plants. For most of the transgenic lines analysed, PTS and/or FPS proteins could be detected in the leaf extracts and the expression level of the recombinant proteins in the leaves was correlated with the levels of terpene (patchoulol) accumulation.

### Example 11: [1-¹³C]-Glucose Labelling for Showing Pathway Used for Terpene Synthesis in Plants of the Present Invention

To determine the biosynthetic origin of the carbon in the patchoulol accumulated, labbeling experiment was conducted and plants of the present invention were compared to plant engineered for plastidic production of patchoulol (PCT/IB2006/051198). Seeds of 8PTS10 (transgenic plant lines expressing PTS in the cytosol) and 2tpPTS+tpFPS12 (plastidic) were germinated on solid MS medium with the addition of hygromycin (15 mg/l).

For GC-MS analysis, thirty 4-week-old seedlings were subsequently transferred to 25ml flasks containing 5 ml of liquid MS medium without sucrose and incubated with gentle shaking (125 rpm) in the dark. One day later, 100 mg of [1-¹³C]-glucose (Sigma) was added into media and plant material collected at daily intervals. For GC analysis, 100 mg samples were extracted as described in Example 7.

For NMR analysis, 1000 seedlings of above two transgenic lines were used, with 100 seedling grown in a 250 ml flask containing 50 ml of liquid MS medium with the addition of 1 g [1-¹³C]-glucose. All labeled seedlings were collected after one week and extracted as above. Concentrated extracts (3 ml) were then purified by preparative TLC separations using silica TLC plates and hexane: ethylacetate (9:1) as the developing solvent.

250 µg of purified ¹³C-labeled patchoulol from line 2tpPTS+tpFPS12 and 500 µg from 8PTS10 were subsequently analyzed by 13C-NMR (750 mHz, CDCl₃ by Bob Coates in the Chemistry Department, University of Illinois, Champaign, IL). Carbon positions and enrichments were assigned relative to un-labeled patchoulol purified from control plants.

Figure 13A shows the predicted ¹³C labelling patterns in patchoulol synthesised from IPP emerging from the MVA and the MEP pathway, respectively, in transgenic seedlings fed on [1-¹³C]-glucose. Patchoulol synthesised from IPP of the MVA pathway is predicted to have 9 ¹³C-carbons at positions 1, 3, 5, 7, 9, 12, 13 14 and 15, whereas its counterpart from the MEP pathway is expected to have only 6 ¹³C-carbons, namely art positions 2, 6, 8, 12, 13 and 15.

Results of GC-MS analysis are shown in Figure 13B, in which Figure b shows MS parent ions for patchoulol synthesised by plants fed ¹²C-glucose (control) and Figures c and d plants fed on ¹³C-glucose producing patchoulol by the (cytosolic) MVA and (plastidic) MEP pathway, respectively. It can be seen that patcholoul emerging from plants fed on ¹³C have heavier ions, with the effect being less pronounced with the plastidic patchoulol: the mass of the major parent ion is shift from 222 to 225, and an M+8 mass parent ion can be observed when the cytosolic pathway is engineered; and the mass of the major parent ion is shifted to 224 and an M+6 mass parent ion can be observed when the chloroplast pathway is engineered. These observation are consistent with the labelling prediction. Results of NRM-analysis are shown in Table 2 below.

**Table 2: ¹³C enrichment into pachoulol synthesized by the cytosoloic MVA and plastidic MEP pathways from 1-C¹³-glucose**

| Carbon atom | Dc (ppm) | % ¹³C | |
|---|---|---|---|
| | | 8PTS-10 | 2tpPTS+tpFPS-12 |
| 1 | 74.9 | 13.0 | 0.0 |
| 2 | 33.1 | 3.0 | 13.8 |
| 3 | 28.9 | 21.8 | 4.2 |
| 4 | 28.1 | 2.9 | 2.7 |
| 5 | 43.8 | 19.1 | 2.4 |
| 6 | 24.5 | 22.2* | 18.4 |
| 7 | 39.4 | 19.3 | 2.7 |
| 8 | 24.9 | 2.9 | 17.3 |
| 9 | 29.1 | 17.1 | 3.6 |
| 10 | 37.8 | 1.6 | 2.0 |
| 11 | 40.3 | 1.4 | 4.0 |
| 12 | 18.8 | 22.9 | 19.1 |
| 13 | 20.9 | 20.2 | 17.9 |
| 14 | 27.3 | 22.0 | 6.1 |
| 15 | 24.7 | 15.8 | 13.8 |

| | | | |
|---|---|---|---|
| *signal due to contaminate | | | |

In accordance with predicted labelling patterns shown in Figure 13, Table 2 shows that in patchoulol from plants having PTS expressed in the cytosol, carbon atoms 1, 3, 5, 7, 9, 12, 13, 14 and 15 are enriched with 13C. The labelling thus shows that the sesquiterpene is actually synthesised from IPP resulting from the MVA-pathway and that there is now contribution of the plastidic pathway.

### Example 12: Engineering of the cytosolic monoterpene biosynthesis in plants.

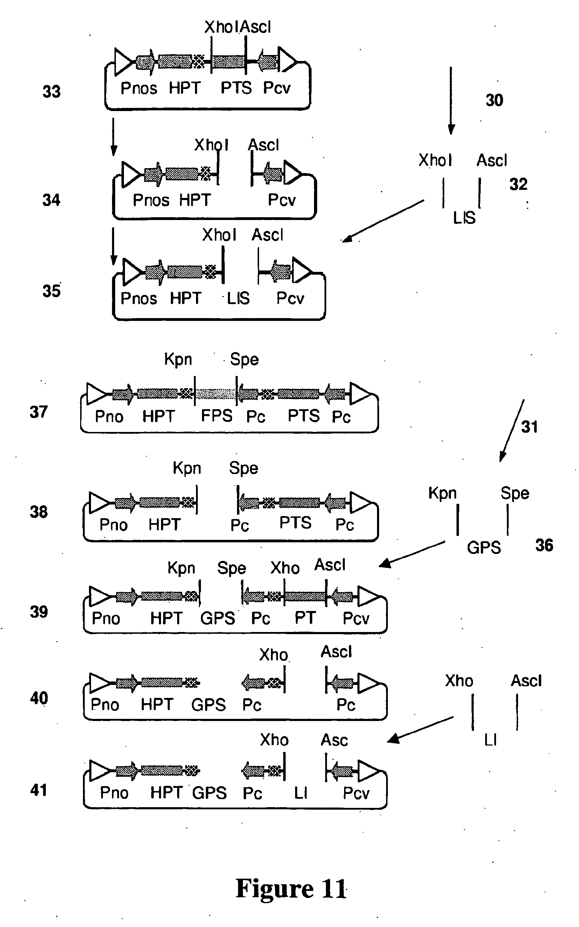

The expression vectors shown in Fig. 7 were generated by substitution cloning of a citrus limonene synthase gene (LIS) (GenBank accession AF514287) for the PTS gene, and a geranyl diphosphate synthase gene (GPS) from Arabidopsis thaliana (GenBank accession ATH17376) for the FPS gene in the original PTS and FPS expression vectors as illustrated in Figure 11. The LIS and GPS genes were obtained by a reverse tmnscription-PCR amplification of the respective genes from RNA isolated from lemon fruits or Arabidopsis plants, respectively. The RNA were extracted using the RNA-Trizol (invitrogen) reagent according to the manufacturer protocol and converted to first strand cDNA using a reverse transcriptase and an oligodT primer. The PCR amplifications were performed using the primers combination AtGPS-SpeF and AtGPS-KpnR for the amplification of GPS and the primers combination LIS-AscF and LIS-XhoR for the amplification of LIS (**30, 31**). These primers were designed to remove the nucleotidic sequence encoding plastid targeting signal, to introduce a start codon and to introduce restriction enzyme recognition sites. The PCR products containing the LIS gene (**32**) was digested with the XhoI and AscI restriction enzymes and ligated in the corresponding site of the original PTS expression vector (**33**) digested with the same enzymes (**34**). The resulting vector (**35**) was used for LIS expression in plants. For the construction of the LIS and GPS expression vector, the PCR product containing the GPS gene (**36**), obtained as described above, was digested with the KpnI and SpeI restriction enzymes and ligated in the PTS+FPS expression vector (**37**) digested with the same enzymes (**38**) to obtain the vector **39** (Figure 11). The PCR product containing the LIS gene and digested with the XhoI and AscI restriction enzymes (32) was obtained as described above and was ligated in the vector **40** first digested with the same enzymes (**42**). The resulting vector (**41**) was used to express LIS and GPS in plants.

Plants were transformed according to Example 6 and evaluated for limonene accumulation according to Example 7. In Figure 15 it can be seen that limonene was accumulated in the plant engineered for the cytosolic pathway and that for some plants coexpression of GPS with LIS increased the accumulation of limonene.

### References

Ausubel FM, Brent R, Kingston RE, Moore DD, Seidman JG, Smith JA, Struhl K (1987) Current Protocols in Molecular Biology.
Benfey PN, Chua NH (1990) The Cauliflower Mosaic Virus-35s Promoter - Combinatorial Regulation Of Transcription In Plants. Science 250: 959-966
Chappell J, Wolf F, Proulx J, Cuellar R, Saunders C (1995) Is The Reaction Catalyzed By 3-Hydroxy-3-Methylglutaryl Coenzyme-A Reductase A Rate-Limiting Step For Isoprenoid Biosynthesis In Plants. Plant Physiology 109: 1337-1343
Chin DJ, Gil G, Russell DW, Liscum L, Luskey KL, Basu SK, Okayama H, Berg P, Goldstein JL, Brown MS (1984) Nucleotide-Sequence Of 3-Hydroxy-3-Methyl-Glutaryl Coenzyme-A Reductase, A Glycoprotein Of Endoplasmic-Reticulum. Nature 308: 613-617
Frey PM, Scharer-Hernandez NG, Futterer J, Potrykus I, Puonti-Kaerlas J (2001) Simultaneous analysis of the bidirectional African cassava mosaic virus promoter activity using two different luciferase genes. Virus Genes 22: 231-242
Hajdukiewicz P, Svab Z, Maliga P (1994) The Small, Versatile Ppzp Family Of Agrobacterium Binary Vectors For Plant Transformation. Plant Molecular Biology 25: 989-994
Hartley JL, Temple GF, Brasch MA (2000) DNA cloning using in vitro site-specific recombination. Genome Research 10: 1788-1795
Horsch RB, Fry JE, Hoffmann NL, Eichholtz D, Rogers SG, Fraley RT (1985) A Simple And General-Method For Transferring Genes Into Plants. Science 227: 1229-1231 Lucker et al (2001) Expression of Clarkia S-linalool synthase in transgenic petunia plants results in the accumulation of S-linalyl-beta-D-glucopyranoside; Plant J. (4):315-24
Mau CJ, West CA (1994) Cloning of casbene synthase cDNA: evidence for conserved structural features among terpenoid cyclases in plants Proc Natl Acad Sci USA; 91(18):8497-501.
Mersereau M, Pazour GJ, Das A (1990) Efficient Transformation Of Agrobacterium-Tumefaciens By Electroporation. Gene 90: 149-151
Murashige T, Skoog F (1962) A Revised Medium For Rapid Growth And Bio Assays With Tobacco Tissue Cultures. Physiologia Plantarum 15: 473-&
Ohara et al (2003) Limonene production in tobacco with Perilla limonene synthase cDNA. J. Exp. Bot.
Peters et al (2000) Biochemistry; 39(50): 15592-602
Sambrook J, Fritsch EF, Maniatis T (1989) Molecular Cloning - a laboratory manual (2nd Edition). Cold Spring Harbor Press, Cold Spring Harbor, NY
Schardl CL, Byrd AD, Benzion G, Altschuler MA, Hildebrand DF, Hunt AG (1987) Design And Construction Of A Versatile System For The Expression Of Foreign Genes In Plants. Gene 61: 1-11
Shirai, K., Masuda, K. and Oosawa, K. 2002. Cloning and Sequencing of Ent-kKaurene Synthase CDNA from Cucumber. Acta Hort. (ISHS) 588:317-320
Takahashi S, Zhao Y, O'Maille PE, Greenhagen BT, Noel JP, Coates RM, Chappell J (2005) Kinetic and molecular analysis of 5-epiaristolochene-1,3-dihydroxylase, a cytochrome P450 enzyme catalyzing successvie hydroxylations of sesquiterpenes. Journal of Biological Chemistry 280: 3686-3696
Tarshis LC, Yan MJ, Poulter CD, Sacchettini JC (1994) Crystal-Structure Of Recombinant Farnesyl Diphosphate Synthase At 2.6-Angstrom Resolution. Biochemistry 33: 10871-10877

## Claims

1. A plant comprising and expressing a transgene encoding a HMG-CoA reductase (HMGR), a transgene encoding a terpene synthase (TS) and a transgene encoding a prenyltransferase (PRT), in which the genes encoding the HMGR, the TS, and the PRT are nuclear genes devoid of a plastid targeting sequence.

2. The plant of claim 1, in which the TS is a mono-, and/or a sesquiterpene synthase.

3. The plant of claim 1 or 2, in which the gene encoding a PRT is a geranyl-diphosphate synthase (GPS) and/or a farnesyl-diphosphate synthase (FPS) encoding gene.

4. The plant of any of the preceding claims, which accumulates, when compared to a native plant not comprising the transgenes, at least 1.2 times more of a terpene than can be synthesised by the TS encoded by the transgene.

5. The plant of any of the preceding claims, which accumulates at least 400 ng/g of fresh leaf of a terpene that can be synthesised by the TS encoded by the transgene and/or additional gene.

6. A method for preparing a transformed plant, the method comprising the steps of
- transforming plant material to comprise additional genes encoding a HMGR, a TS and a PRT, said genes being nuclear genes devoid of a plastid targeting sequence; and
- regenerating transformed plants from said plant material.

7. The method of claim 6, **characterized in that** said transformed plant has an altered terpene content.

8. A method for producing a terpene, the method comprising the step of isolating the terpene from a plant comprising and expressing a transgene encoding a HMG-CoA reductase (HMGR), a transgene encoding a terpene synthase (TS) and a transgene encoding a prenyltransferase (PRT), in which the genes encoding the HMGR, the TS, and the PRT are nuclear genes devoid of a plastid targeting sequence.

## Patentansprüche

1. Pflanze, umfassend und exprimierend ein Transgen, codierend eine HMG-CoA-Reduktase (HMGR), ein Transgen, codierend eine Terpensynthase (TS), und ein Transgen, codierend eine Prenyltransferase (PRT), in welcher die Gene, codierend die HMGR, die TS und die PRT, nukleäre Gene, frei von einer Plastidzielsequenz, sind.

2. Pflanze nach Anspruch 1, in welcher die TS eine Mono- und/oder eine Sesquiterpensynthase ist.

3. Pflanze nach Anspruch 1 oder 2, in welcher das Gen, codierend eine PRT, ein Geranyl-Diphosphat-Synthase (GPS) und/oder ein Farnesyl-Diphosphat-Synthase (FPS) codierendes Gen ist.

4. Pflanze nach einem der vorhergehenden Ansprüche, welche, wenn verglichen mit einer nativen Pflanze, nicht umfassend die Transgene, mindestens 1,2 mal mehr von einem Terpen akkumuliert, als durch die TS, codiert durch das Transgen, synthetisiert werden kann.

5. Pflanze nach einem der vorhergehenden Ansprüche, welche mindestens 400 ng/g von frischem Blatt von einem Terpen akkumuliert, das durch die TS, codiert durch das Transgen und/oder zusätzliches Gen, synthetisiert werden kann.

6. Verfahren zum Herstellen einer transformierten Pflanze, wobei das Verfahren die Schritte umfaßt,
- Pflanzenmaterial zu transformieren, um zusätzliche Gene, codierend eine HMGR, eine TS und eine PRT, zu umfassen, wobei die Gene nukleäre Gene, frei von einer Plastidzielsequenz, sind; und
- transformierte Pflanzen aus dem Pflanzenmaterial zu regenerieren.

7. Verfahren nach Anspruch 6, **dadurch gekennzeichnet, daß** die transformierte Pflanze einen veränderten Terpengehalt hat.

8. Verfahren zum Erzeugen eines Terpens, wobei das Verfahren den Schritt umfaßt, das Terpen aus einer Pflanze, umfassend und exprimierend ein Transgen, codierend eine HMG-CoA-Reduktase (HMGR), ein Transgen, codierend eine Terpensynthase (TS), und ein Transgen, codierend eine Prenyltransferase (PRT), zu isolieren, in welcher die Gene, codierend die HMGR, die TS und die PRT, nukleäre Gene, frei von einer Plastidzielsequenz, sind.

## Revendications

1. Plante comprenant et exprimant un transgène codant pour une HMG-CoA réductase (HMGR), un transgène codant pour une terpène synthase (TS) et un transgène codant pour une prényltransférase (PRT), dans laquelle les gènes codant pour la HMGR, la TS et la PRT sont des gènes nucléaires dépourvus de séquence ciblant les plastides.

2. Plante selon la revendication 1, dans laquelle la TS est une mono-, et/ou une sesquiterpène synthase.

3. Plante selon la revendication 1 ou 2, dans laquelle le gène codant pour une PRT est un gène codant pour une géranyl-diphosphate synthase (GPS) et/ou pour une farnésyl-diphosphate synthase (FPS).

4. Plante selon l'une quelconque des revendications précédentes, qui accumule, comparativement à une plante native ne comprenant pas les transgènes, au moins 1,2 fois plus d'un terpène qui peut être synthétisé par la TS codée par le transgène.

5. Plante selon l'une quelconque des revendications précédentes, qui accumule au moins 400 ng/g de feuille fraîche d'un terpène qui peut être synthétisé par la TS codée par le transgène et/ou un gène additionnel.

6. Procédé de préparation d'une plante transformée, ledit procédé comprenant les étapes consistant à
- transformer une matière végétale pour qu'elle comprenne des gènes additionnels codant pour une HMGR, une TS et une PRT, lesdits gènes étant des gènes nucléaires dépourvus de séquence ciblant les plastides; et
- régénérer des plantes transformées à partir de ladite matière végétale.

7. Procédé selon la revendication 6, **caractérisé en ce que** ladite plante transformée a une teneur en terpène modifiée.

8. Procédé de production d'un terpène, ledit procédé comprenant l'étape consistant à isoler le terpène d'une plante comprenant et exprimant un transgène codant pour une HMG-CoA réductase (HMGR), un transgène codant pour une terpène synthase (TS) et un transgène codant pour une prényltransférase (PRT), dans lequel les gènes codant pour la HMGR, la TS et la PRT sont des gènes nucléaires dépourvus de séquence ciblant les plastides.


### SEQUENCE LISTING

<110> University of Kentucky Research Foundation Wu, Shuiqin
   Chappell, Joseph
   Schalk, Michel
   Clark, Anthony
<120> Transformed Plants Accumulating Mono- and Sesquiterpenes
<130> 6510PROV-PCT
<160> 57
<170> PatentIn version 3.3
<210> 1
   <211> 1659
   <212> DNA
   <213> Progostemon cablin
<400> 1
   atggagttgt atgcccaaag tgttggagtg ggtgctgctt ctcgtcctct tgcgaatttt     60
   catccatgtg tgtggggaga caaattcatt gtctacaacc cacaatcatg ccaggctgga    120
   gagagagaag aggctgagga gctgaaagtg gagctgaaaa gagagctgaa ggaagcatca    180
   gacaactaca tgcggcaact gaaaatggtg gatgcaatac aacgattagg cattgactat    240
   ctttttgtgg aagatgttga tgaagctttg aagaatctgt ttgaaatgtt tgatgctttc    300
   tgcaagaata atcatgacat gcacgccact gctctcagct ttcgccttct cagacaacat    360
   ggatacagag tttcatgtga agtttttgaa aagtttaagg atggcaaaga tggatttaag    420
   gttccaaatg aggatggagc ggttgcagtc cttgaattct tcgaagccac gcatctcaga    480
   gtccatggag aagacgtcct tgataatgct tttgacttca ctaggaacta cttggaatca    540
   gtctatgcaa ctttgaacga tccaaccgcg aaacaagtcc acaacgcatt gaatgagttc    600
   tcttttcgaa gaggattgcc acgcgtggaa gcaaggaagt acatatcaat ctacgagcaa    660
   tacgcatctc atcacaaagg cttgctcaaa cttgctaagc tggatttcaa cttggtacaa    720
   gctttgcaca gaagggagct gagtgaagat tctaggtggt ggaagacttt acaagtgccc    780
   acaaagctat cattcgttag agatcgattg gtggagtcct acttctgggc ttcgggatct    840
   tatttcgaac cgaattattc ggtagctagg atgattttag caaaagggct ggctgtatta    900
   tctcttatgg atgatgtgta tgatgcatat ggtacttttg aggaattaca aatgttcaca    960
   gatgcaatcg aaaggtggga tgcttcatgt ttagataaac ttccagatta catgaaaata   1020
   gtatacaagg cccttttgga tgtgtttgag gaagttgacg aggagttgat caagctaggc   1080
   gcaccatatc gagcctacta tggaaaagaa gccatgaaat acgccgcgag agcttacatg   1140
   gaagaggccc aatggaggga gcaaaagcac aaacccacaa ccaaggagta tatgaagctg   1200
   gcaaccaaga catgtggcta cataactcta ataatattat catgtcttgg agtggaagag   1260
   ggcattgtga ccaaagaagc cttcgattgg gtgttctccc gacctccttt catcgaggct   1320
   acattaatca ttgccaggct cgtcaatgat attacaggac acgagtttga gaaaaaacga   1380
   gagcacgttc gcactgcagt agaatgctac atggaagagc acaaagtggg gaagcaagag   1440
   gtggtgtctg aattctacaa ccaaatggag tcagcatgga aggacattaa tgaggggttc   1500
   ctcagaccag ttgaatttcc aatccctcta ctttatctta ttctcaattc agtccgaaca   1560
   cttgaggtta tttacaaaga gggcgattcg tatacacacg tgggtcctgc aatgcaaaac   1620
   atcatcaagc agttgtacct tcaccctgtt ccatattaa                          1659
<210> 2
   <211> 1137
   <212> DNA
   <213> Gallus gallus
<400> 2
   atgcagcccc atcatcatca taaagagggg cgtatgcata aatttactgg tgtcaatgcc     60
   aagtttcagc aacccgcgtt gaggaacctc agccccgtgg tggttgagag ggagagggag    120
   gagttcgtgg ggttcttccc gcagatcgtc cgcgatctga ccgaggacgg catcggacac    180
   ccggaggtgg gcgacgctgt ggcgcggctg aaggaggtgc tgcaatacaa cgctcccggt    240
   gggaaatgca accgtgggct gacggtggtg gctgcgtacc gggagctgtc ggggccgggg    300
   cagaaggatg ctgagagcct gcggtgcgcg ctggccgtgg gttggtgcat cgagttgttc    360
   caggccttct tcctggtggc tgatgatatc atggatcagt ccctcacgcg ccgggggcag    420
   ctgtgttggt ataagaagga gggggtcggt ttggatgcca tcaacgactc cttcctcctc    480
   gagtcctctg tgtacagagt gctgaagaag tactgcaggc agcggccgta ttacgtgcat    540
   ctgttggagc tcttcctgca gaccgcctac cagactgagc tcgggcagat gctggacctc    600
   atcacagctc ccgtctccaa agtggatttg agtcacttca gcgaggagag gtacaaagcc    660
   atcgttaagt acaagactgc cttctactcc ttctacctac ccgtggctgc tgccatgtat    720
   atggttggga tcgacagtaa ggaagaacac gagaatgcca aagccatcct gctggagatg    780
   ggggaatact tccagatcca ggatgattac ctggactgct ttggggaccc ggcgctcacg    840
   gggaaggtgg gcaccgacat ccaggacaat aaatgcagct ggctcgtggt gcagtgcctg    900
   cagcgcgtca cgccggagca gcggcagctc ctggaggaca actacggccg taaggagccc    960
   gagaaggtgg cgaaggtgaa ggagctgtat gaggccgtgg ggatgagggc tgcgttccag   1020
   cagtacgagg agagcagcta ccggcgcctg caggaactga tagagaagca ctcgaaccgc   1080
   ctcccgaagg agatcttcct cggcctggca cagaagatct acaaacgcca gaaatga      1137
<210> 3
   <211> 2802
   <212> DNA
   <213> Artificial
<220>
   <223> DNA encodes fusion protein FPS-PTS
<400> 3
   atgcagcccc atcatcatca taaagagggg cgtatgcata aatttactgg tgtcaatgcc     60
   aagtttcagc aacccgcgtt gaggaacctc agccccgtgg tggttgagag ggagagggag    120
   gagttcgtgg ggttcttccc gcagatcgtc cgcgatctga ccgaggacgg catcggacac    180
   ccggaggtgg gcgacgctgt ggcgcggctg aaggaggtgc tgcaatacaa cgctcccggt    240
   gggaaatgca accgtgggct gacggtggtg gctgcgtacc gggagctgtc ggggccgggg    300
   cagaaggatg ctgagagcct gcggtgcgcg ctggccgtgg gttggtgcat cgagttgttc    360
   caggccttct tcctggtggc tgatgatatc atggatcagt ccctcacgcg ccgggggcag    420
   ctgtgttggt ataagaagga gggggtcggt ttggatgcca tcaacgactc cttcctcctc    480
   gagtcctctg tgtacagagt gctgaagaag tactgcaggc agcggccgta ttacgtgcat    540
   ctgttggagc tcttcctgca gaccgcctac cagactgagc tcgggcagat gctggacctc    600
   atcacagctc ccgtctccaa agtggatttg agtcacttca gcgaggagag gtacaaagcc    660
   atcgttaagt acaagactgc cttctactcc ttctacctac ccgtggctgc tgccatgtat    720
   atggttggga tcgacagtaa ggaagaacac gagaatgcca aagccatcct gctggagatg    780
   ggggaatact tccagatcca ggatgattac ctggactgct ttggggaccc ggcgctcacg    840
   gggaaggtgg gcaccgacat ccaggacaat aaatgcagct ggctcgtggt gcagtgcctg    900
   cagcgcgtca cgccggagca gcggcagctc ctggaggaca actacggccg taaggagccc    960
   gagaaggtgg cgaaggtgaa ggagctgtat gaggccgtgg ggatgagggc tgcgttccag   1020
   cagtacgagg agagcagcta ccggcgcctg caggaactga tagagaagca ctcgaaccgc   1080
   ctcccgaagg agatcttcct cggcctggca cagaagatct acaaacgcca gaaaggcgcg   1140
   ccgatggagt tgtatgccca aagtgttgga gtgggtgctg cttctcgtcc tcttgcgaat   1200
   tttcatccat gtgtgtgggg agacaaattc attgtctaca acccacaatc atgccaggct   1260
   ggagagagag aagaggctga ggagctgaaa gtggagctga aaagagagct gaaggaagca   1320
   tcagacaact acatgcggca actgaaaatg gtggatgcaa tacaacgatt aggcattgac   1380
   tatctttttg tggaagatgt tgatgaagct ttgaagaatc tgtttgaaat gtttgatgct   1440
   ttctgcaaga ataatcatga catgcacgcc actgctctca gctttcgcct tctcagacaa   1500
   catggataca gagtttcatg tgaagttttt gaaaagttta aggatggcaa agatggattt   1560
   aaggttccaa atgaggatgg agcggttgca gtccttgaat tcttcgaagc cacgcatctc   1620
   agagtccatg gagaagacgt ccttgataat gcttttgact tcactaggaa ctacttggaa   1680
   tcagtctatg caactttgaa cgatccaacc gcgaaacaag tccacaacgc attgaatgag   1740
   ttctcttttc gaagaggatt gccacgcgtg gaagcaagga agtacatatc aatctacgag   1800
   caatacgcat ctcatcacaa aggcttgctc aaacttgcta agctggattt caacttggta   1860
   caagctttgc acagaaggga gctgagtgaa gattctaggt ggtggaagac tttacaagtg   1920
   cccacaaagc tatcattcgt tagagatcga ttggtggagt cctacttctg ggcttcggga   1980
   tcttatttcg aaccgaatta ttcggtagct aggatgattt tagcaaaagg gctggctgta   2040
   ttatctctta tggatgatgt gtatgatgca tatggtactt ttgaggaatt acaaatgttc   2100
   acagatgcaa tcgaaaggtg ggatgcttca tgtttagata aacttccaga ttacatgaaa   2160
   atagtataca aggccctttt ggatgtgttt gaggaagttg acgaggagtt gatcaagcta   2220
   ggcgcaccat atcgagccta ctatggaaaa gaagccatga aatacgccgc gagagcttac   2280
   atggaagagg cccaatggag ggagcaaaag cacaaaccca caaccaagga gtatatgaag   2340
   ctggcaacca agacatgtgg ctacataact ctaataatat tatcatgtct tggagtggaa   2400
   gagggcattg tgaccaaaga agccttcgat tgggtgttct cccgacctcc tttcatcgag   2460
   gctacattaa tcattgccag gctcgtcaat gatattacag gacacgagtt tgagaaaaaa   2520
   cgagagcacg ttcgcactgc agtagaatgc tacatggaag agcacaaagt ggggaagcaa   2580
   gaggtggtgt ctgaattcta caaccaaatg gagtcagcat ggaaggacat taatgagggg   2640
   ttcctcagac cagttgaatt tccaatccct ctactttatc ttattctcaa ttcagtccga   2700
   acacttgagg ttatttacaa agagggcgat tcgtatacac acgtgggtcc tgcaatgcaa   2760
   aacatcatca agcagttgta ccttcaccct gttccatatt aa                      2802
<210> 4
   <211> 1668
   <212> DNA
   <213> Citrus limon
<400> 4
   atgaggagat cagcaaacta ccaaccttca atttgggacc acgatttttt gcagtcattg 60
   aatagcaact atacggatga agcatacaaa agacgagcag aagagctgag gggaaaagtg 120
   aagatagcga ttaaggatgt aatcgagcct ctggatcagt tggagctgat tgataacttg 180
   caaagacttg gattggctca tcgttttgag actgagatta ggaacatatt gaataatatc 240
   tacaacaata ataaagatta taattggaga aaagaaaatc tgtatgcaac ctcccttgaa 300
   ttcagactac ttagacaaca tggctatcct gtttctcaag aggttttcaa tggttttaaa 360
   gacgaccagg gaggcttcat ttgtgatgat ttcaagggaa tactgagctt gcatgaagct 420
   tcgtattaca gcttagaagg agaaagcatc atggaggagg cctggcaatt tactagtaaa 480
   catcttaaag aagtgatgat cagcaagaac atggaagagg atgtatttgt agcagaacaa 540
   gcgaagcgtg cactggagct ccctctgcat tggaaagtgc caatgttaga ggcaaggtgg 600
   ttcatacaca tttatgagag aagagaggac aagaaccacc ttttacttga gctcgctaag 660
   atggagttta acactttgca ggcaatttac caggaagaac taaaagaaat ttcagggtgg 720
   tggaaggata caggtcttgg agagaaattg agctttgcga ggaacaggtt ggtagcgtcc 780
   ttcttatgga gcatggggat cgcgtttgag cctcaattcg cctactgcag gagagtgctc 840
   acaatctcga tagccctaat tacagtgatt gatgacattt atgatgtcta tggaacattg 900
   gatgaacttg agatattcac tgatgctgtt gagaggtggg acatcaatta tgctttgaag 960
   caccttccgg gctatatgaa aatgtgtttt cttgcgcttt acaactttgt taatgaattt 1020
   gcttattacg ttctcaaaca acaggatttt gatttgcttc tgagcataaa aaatgcatgg 1080
   cttggcttaa tacaagccta cttggtggag gcgaaatggt accatagcaa gtacacaccg 1140
   aaactggaag aatacttgga aaatggattg gtatcaataa cgggcccttt aattataacg 1200
   atttcatatc tttctggtac aaatccaatc attaagaagg aactggaatt tctagaaagt 1260
   aatccagata tagttcactg gtcatccaag attttccgtc tgcaagatga tttgggaact 1320
   tcatcggacg agatacagag aggggatgtt ccgaaatcaa tccagtgtta catgcatgaa 1380
   actggtgcct cagaggaagt tgctcgtcaa cacatcaagg atatgatgag acagatgtgg 1440
   aagaaggtga atgcatacac agccgataaa gactctccct tgactggaac aactactgag 1500
   ttcctcttga atcttgtgag aatgtcccat tttatgtatc tacatggaga tgggcatggt 1560
   gttcaaaacc aagagactat cgatgtcggt tttacattgc tttttcagcc cattcccttg 1620
   gaggacaaac acatggcttt cacagcatct cctggcacca aaggctga 1668
<210> 5
   <211> 1035
   <212> DNA
   <213> Arabidopsis thaliana
<400> 5
   atggaggagc ttgacccatt ttcgcttgtt gccgatgagc tgtcacttct tagtaataag     60
   ttgagagaga tggtacttgc cgaggttcca aagcttgcct ctgctgctga gtacttcttc    120
   aaaaggggtg tgcaaggaaa acagtttcgt tcaactattt tgctgctgat ggcgacagct    180
   ctggatgtac gagttccaga agcattgatt ggggaatcaa cagatatagt cacatcagaa    240
   ttacgcgtaa ggcaacgggg tattgctgaa atcactgaaa tgatacacgt cgcaagtcta    300
   ctgcacgatg atgtcttgga tgatgccgat acaaggcgtg gtgttggttc cttaaatgtt    360
   gtaatgggta acaagatgtc ggtattagca ggagacttct tgctctcccg ggcttgtggg    420
   gctctcgctg ctttaaagaa cacagaggtt gtagcattac ttgcaactgc tgtagaacat    480
   cttgttaccg gtgaaaccat ggagataact agttcaaccg agcagcgtta tagtatggac    540
   tactacatgc agaagacata ttataagaca gcatcgctaa tctctaacag ctgcaaagct    600
   gttgccgttc tcactggaca aacagcagaa gttgccgtgt tagcttttga gtatgggagg    660
   aatctgggtt tagcattcca attaatagac gacattcttg atttcacggg cacatctgcc    720
   tctctcggaa agggatcgtt gtcagatatt cgccatggag tcataacagc cccaatcctc    780
   tttgccatgg aagagtttcc tcaactacgc gaagttgttg atcaagttga aaaagatcct    840
   aggaatgttg acattgcttt agagtatctt gggaagagca agggaataca gagggcaaga    900
   gaattagcca tggaacatgc gaatctagca gcagctgcaa tcgggtctct acctgaaaca    960
   gacaatgaag atgtcaaaag atcgaggcgg gcacttattg acttgaccca tagagtcatc   1020
   accagaaaca agtga                                                    1035
<210> 6
   <211> 27
   <212> DNA
   <213> Artificial
<220>
   <223> Primer (Attp1-SstI-FW)
<400> 6
   agtcacgagc tcgtaaaacg acggcca 27
<210> 7
   <211> 23
   <212> DNA
   <213> Artificial
<220>
   <223> Primer (Attp2-SphI-RV)
<400> 7
   gcatgccagg aaacagctat gac 23
<210> 8
   <211> 26
   <212> DNA
   <213> Artificial
<220>
   <223> Primer (HPT-NotI-FD)
<400> 8
   gcggccgcat gaaaaagcct gaactc 26
<210> 9
   <211> 25
   <212> DNA
   <213> Artificial
<220>
   <223> Primer (HPT-Xba1-RV)
<400> 9
   tctagataat tcgggggatc tggat 25
<210> 10
   <211> 26
   <212> DNA
   <213> Artificial
<220>
   <223> Primer (TB-SphI- FD)
<400> 10
   gtggttggca tgcacataca aatgga 26
<210> 11
   <211> 26
   <212> DNA
   <213> Artificial
<220>
   <223> Primer (TB-NotI-RV
<400> 11
   gcggccgcgc gaaacgatcc agatcc 26
<210> 12
   <211> 28
   <212> DNA
   <213> Artificial
<220>
   <223> Primer (HPT-SphI-RV)
<400> 12
   ggggcatgct aattcggggg atctggat 28
<210> 13
   <211> 56
   <212> DNA
   <213> Artificial
<220>
   <223> Primer (CSMV-ATTB1-SGFI-FD)
<400> 13
   ggggacaagt ttgtacaaaa aagcaggctg cgatcgccct atgttcaaaa atgaag 56
<210> 14
   <211> 31
   <212> DNA
   <213> Artificial
<220>
   <223> Primer (CSMV-ECORI-RV)
<400> 14
   tacgaattca caaatttctc tgaagttgta t 31
<210> 15
   <211> 31
   <212> DNA
   <213> Artificial
<220>
   <223> Primer (Tnos-XhoI-FW)
<400> 15
   cggatgctcg aggatcgttc aaacatttgg c 31
<210> 16
   <211> 49
   <212> DNA
   <213> Artificial
<220>
   <223> Primer (Tnos-attB2-RV)
<400> 16
   ggggaccact ttgtacaaga aagctgggtg atctagtaac atagatgac 49
<210> 17
   <211> 35
   <212> DNA
   <213> Artificial
<220>
   <223> Primer (His-EcoF)
<400> 17
   aattcggcgc gccatgcatc atcatcatca tcacg 35
<210> 18
   <211> 35
   <212> DNA
   <213> Artificial
<220>
   <223> Primer (His-EcoR)
<400> 18
   aattcgtgat gatgatgatg atgcatggcg cgccg 35
<210> 19
   <211> 44
   <212> DNA
   <213> Artificial
<220>
   <223> Primer (mHisPTS-F)
<400> 19
   cttcagagaa atttgtgaat tatacatcat catcatcatc acgg 44
<210> 20
   <211> 44
   <212> DNA
   <213> Artificial
<220>
   <223> Primer (mHisPTS-R)
<400> 20
   ccgtgatgat gatgatgatg tataattcac aaatttctct gaag 44
<210> 21
   <211> 31
   <212> DNA
   <213> Artificial
<220>
   <223> Primer (TP-ASCF)
<400> 21
   ttggcgcgcc tatggcttcc tctatgctct c 31
<210> 22
   <211> 31
   <212> DNA
   <213> Artificial
<220>
   <223> Primer (TP-ASCR)
<400> 22
   ttggcgcgcc cttcatgcag ctagatcttc c 31
<210> 23
   <211> 31
   <212> DNA
   <213> Artificial
<220>
   <223> Primer (PCaMV-XbaI-FW)
<400> 23
   tcagtttcta gacatggagt caaagattca a 31 <210> 24
   <211> 27
   <212> DNA
   <213> Artificial
<220>
   <223> Primer (PCaMV-SpeI-RV)
<400> 24
   accatgacta gtcccccgtg ttctctc 27
<210> 25
   <211> 28
   <212> DNA
   <213> Artificial
<220>
   <223> Primer (FPS-SpeI-FW)
<400> 25
   actagtatgc aattcttcag cttggttt 28
<210> 26
   <211> 33
   <212> DNA
   <213> Artificial
<220>
   <223> Primer (FPS-KpnI-RV)
<400> 26
   cggggtacct tactcccgag aaggttgata agg 33
<210> 27
   <211> 28
   <212> DNA
   <213> Artificial
<220>
   <223> Primer (Tnos-KpnI-FW)
<400> 27
   cggggtaccg atcgttcaaa catttggc 28
<210> 28
   <211> 21
   <212> DNA
   <213> Artificial
<220>
   <223> Primer (Tnos-attB2-RV3)
<400> 28
   gagctcgggg accactttgt a 21
<210> 29
   <211> 56
   <212> DNA
   <213> Artificial
<220>
   <223> Primer (CsMV-attB1-Sgf I-FD)
<400> 29
   ggggacaagt ttgtacaaaa aagcaggctg cgatcgccct atgttcaaaa atgaag 56
<210> 30
   <211> 28
   <212> DNA
   <213> Artificial
<220>
   <223> Primer (TNOS-XbaI-RV)
<400> 30
   gctctagaga tctagtaaca tagatgac 28
<210> 31
   <211> 30
   <212> DNA
   <213> Artificial
<220>
   <223> Primer (TP-SpeFW)
<400> 31
   ggggactagt atggcttcct ctatgctctc 30
<210> 32
   <211> 31
   <212> DNA
   <213> Artificial
<220>
   <223> Primer (TP-SpeRV)
<400> 32
   ggggactagt cttcatgcag ctagatcttc c 31
<210> 33
   <211> 34
   <212> DNA
   <213> Artificial
<220>
   <223> Primer (PTS-AscF)
<400> 33
   ggggagctcg gcgcgccgat ggagttgtat gccc 34
<210> 34
   <211> 31
   <212> DNA
   <213> Artificial
<220>
   <223> Primer (PTS-XhoR)
<400> 34
   ggggctcgag ttaatatgga acagggtgaa g 31
<210> 35
   <211> 35
   <212> DNA
   <213> Artificial
<220>
   <223> Primer (FPP-SpeFW:)
<400> 35
   ggggactagt atgcagcccc atcatcatca taaag 35
<210> 36
   <211> 31
   <212> DNA
   <213> Artificial
<220>
   <223> Primer (FPP-KpnRV)
<400> 36
   cggggtacct catttctggc gtttgtagat c 31
<210> 37
   <211> 30
   <212> DNA
   <213> Artificial
<220>
   <223> Primer (FPS-AscF:)
<400> 37
   ttggcgcgcc tatgcagccc catcatcatc 30
<210> 38
   <211> 30
   <212> DNA
   <213> Artificial
<220>
   <223> Primer (FPS-AscR:)
<400> 38
   ttggcgcgcc ttttctggcg tttgtagatc 30
<210> 39
   <211> 24
   <212> DNA
   <213> Artificial
<220>
   <223> Primer (PTS-8F:)
<400> 39
   gagtgggtgc tgcttctcgt cctc 24
<210> 40
   <211> 24
   <212> DNA
   <213> Artificial
<220>
   <223> Primer (PTS-160R)
<400> 40
   ctccatggac tctgagatgc gtgg 24
<210> 41
   <211> 26
   <212> DNA
   <213> Artificial
<220>
   <223> Primer (FPP-1F)
<400> 41
   gcagccccat catcatcata aagagg 26
<210> 42
   <211> 23
   <212> DNA
   <213> Artificial
<220>
   <223> Primer (FPP-160R)
<400> 42
   gaggactcga ggaggaagga gtc 23
<210> 43
   <211> 21
   <212> DNA
   <213> Artificial
<220>
   <223> Primer (RBCS-FW)
<400> 43
   atgcaggtgt ggccaccaat t 21
<210> 44
   <211> 21
   <212> DNA
   <213> Artificial
<220>
   <223> Primer (RBCS-RV)
<400> 44
   ttagtagcct tctggcttgt a 21
<210> 45
   <211> 33
   <212> DNA
   <213> Artificial
<220>
   <223> Primer (AtGPS-SpeF)
<400> 45
   ggactagtat ggaggagctt gacccatttt cgc 33
<210> 46
   <211> 31
   <212> DNA
   <213> Artificial
<220>
   <223> Primer (AtGPS-KpnR)
<400> 46
   ggggtacctc acttgtttct ggtgatgact c 31
<210> 47
   <211> 37
   <212> DNA
   <213> Artificial
<220>
   <223> Primer (LIS-AscF)
<400> 47
   gggggcgcgc cgatgaggag atcagcaaac taccaac 37
<210> 48
   <211> 34
   <212> DNA
   <213> Artificial
<220>
   <223> Primer (LIS-XhoR)
<400> 48
   ggggctcgag tcagcctttg gtgccaggag atgc 34
<210> 49
   <211> 7
   <212> PRT
   <213> Artificial
<220>
   <223> Antigenic peptide (PTS46)
<400> 49
   Glu Glu Leu Lys Val Glu Leu
   1 5
<210> 50
   <211> 20
   <212> PRT
   <213> Artificial
<220>
   <223> Antigenic peptide (PTS108)
<400> 50
   His Ala Thr Ala Leu Ser Phe Arg Leu Leu Arg Gln His Gly Tyr Arg
   1 5 10 15
   Val Ser Cys Glu
   20
<210> 51
   <211> 13
   <212> PRT
   <213> Artificial
<220>
   <223> Antigenic peptide (PTS353)
<400> 51
   Asp Glu Glu Leu Ile Lys Leu Gly Ala Pro Tyr Arg Ala
   1 5 10
<210> 52
   <211> 11
   <212> PRT
   <213> Artificial
<220>
   <223> Antigenic peptide (PTS462)
<400> 52
   His Val Arg Thr Ala Val Glu Cys Tyr Met Glu
   1 5 10
<210> 53
   <211> 10
   <212> PRT
   <213> Artificial
<220>
   <223> Antigenic peptide (PTS475)
<400> 53
   Lys Val Gly Lys Gln Glu Val Val Ser Glu
   1               5                   10
<210> 54
   <211> 11
   <212> PRT
   <213> Artificial
<220>
   <223> Antigenic peptide (FPP41)
<400> 54
   Glu Phe Val Gly Phe Phe Pro Gln Ile Val Arg
   1               5                   10
<210> 55
   <211> 18
   <212> PRT
   <213> Artificial
<220>
   <223> Antigenic peptide (FPP59)
<400> 55
   Gly His Pro Glu Val Gly Asp Ala Val Ala Arg Leu Lys Glu Val Leu
   1               5                   10                  15
   Gln Tyr
<210> 56
   <211> 20
   <212> PRT
   <213> Artificial
<220>
   <223> Antigenic peptide (FPP218)
<400> 56
   Tyr Lys Ala Ile Val Lys Tyr Lys Thr Ala Phe Tyr Ser Phe Tyr Leu
   1               5                   10                  15
   Pro Val Ala Ala
               20
<210> 57
<211> 13
   <212> PRT
   <213> Artificial
<220>
   <223> Antigenic peptide (FPP320)
<400> 57
   Pro Glu Lys Val Ala Lys Val Lys Glu Leu Tyr Glu Ala
   1               5                   10